# EUROPEAN PATENT APPLICATION

(11) **EP 1 816 475 A1**
(43) Date of publication of application: **08.08.2007**
(21) Application number: 07090023.8
(22) Date of filing: 22.07.2004
(51) Int. Cl.: G01N 33/574, A61K 47/48, A61K 49/00, G01N 33/533

(54) **Use of cyanine dyes for the diagnosis of disease associated with angiogenesis**

(62) Divisional of application: 04017375.9
(71) Applicant: Bayer Schering Pharma Aktiengesellschaft, 13353 Berlin (DE)
(72) Inventor: Schirner, Michael, 13158 Berlin (DE); Hauff, Peter, 10439 Berlin (DE); Licha, Kai, 14612 Falkensee (DE); Perlitz, Christin, 10115 Berlin (DE)

(57) **Abstract**

This invention relates to the use of conjugates of cyanine dyes with an angiogenesis specific binding component preferably with an EB-D fibronectin specific binding component for the diagnosis of micrometastasis and small proliferative lesions, in particular primary tumors, precancerosis, dysplasia, metaplasia, endometriosis, ocular diseases associated with angiogenesis, psoriasis, psoriatic arthritis and/or rheumatoid arthritis.

## Description

This invention relates to the use of conjugates of cyanine dyes with an angiogenesis specific binding component preferably with an EB-D fibronectin specific binding component for the diagnosis of micrometastasis and small proliferative lesions, in particular primary tumors, precancerosis, dysplasia, metaplasia, endometriosis, ocular diseases associated with angiogenesis, psoriasis, psoriatic arthritis and/or rheumatoid arthritis.

### Background of the Invention

The use of light in medical diagnosis has recently gained importance (see, e.g., Biomedical Photonics Handbook (Editor: T. Vo-Dinh), CRC Press). A wide variety of diagnostic processes are under experimental testing for application in various medical disciplines, e.g. endoscopy, mammography, surgery or gynecology. To this end dyes are fed to the tissue as exogenic contrast media for fluorescence diagnosis and imaging, and here in particular fluorescence dyes with an absorption and fluorescence maximum in the spectral range of 700-900 nm (diagnostic window of tissue), have been used for *in vivo* imaging. Photons of this wavelength are comparatively little absorbed by tissue and can therefore penetrate several centimeters into the tissue before the absorption process (primarily by oxyhemoglobin and deoxyhemoglobin) ends the light transport. Absorption can take place, moreover, by the fluorescence dyes that are introduced into the tissue, but that emit the absorbed energy in the form of longer-wave fluorescence radiation. This fluorescence radiation can be detected spectrally separated and makes possible the localization of dyes and the correlation with molecular structures to which the dye has bonded (see in this respect also Licha, K. (2002) Contrast Agents for Optical Imaging (Review). In: Topics in Current Chemistry - Contrast Agents II (Editor: W. Krause), Volume 222, Springer Heidelberg, pp. 1 - 31.).

Fluorescence dyes from the class of cyanine dyes fall into the category of promising representatives and were synthesized in many different structural widths. In particular, carbocyanines with indocarbocyanine, indodicarbocyanine and indotricarbocyanine skeletons have high extinction coefficients and good fluorescence quantum yields (Licha, K. (2002) *supra,* and the references cited therein).

To achieve a diagnostically significant differentiation between diseased structures and healthy tissue, the dye that is administered must lead to as high a concentration difference between the two tissue types as possible. This can be carried out based on tumor-physiological or morphological properties (blood supply, distribution kinetics, delayed removal, vessel structures) as well as based on molecular properties of the tumor and vessel cell or adjacent tissue. For molecular labeling of disease-specific structures, conjugates that consist of fluorescence dyes with target-affine molecules, such as proteins, peptides, or antibodies, can be used. After injection, a certain portion of these conjugates binds to molecular target structures, such as receptors, cell surface structures or matrix proteins, while the unbonded portion remains diluted or metabolized in the bodily fluids or is excreted from the body. In this way, a higher concentration difference and, thus, a greater image contrast in implementing the fluorescence diagnosis may result (high signal-to-noise ratio).

It has been described that many diseases like, for example, tumors (Folkman J. (1974). Symp. Soc. Dev. Biol. 30:43-52), arthritis (Colville-Nash PR, Scott DL (1992) Ann. Rheum. Dis. 51:919-25), psoriasis (Folkman J. (1972) J. Invest. Dermatol. 59:40-43), ocular diseases (Adamis AP, et al. (1999) Angiogenesis, 3:9-14) are associated with angiogenesis. The various diseases associated with angiogenesis are reviewed in, for example, Longo R, et al. (2002) Angiogenesis 5:237-56. On the other hand the formation of new blood vessels rarely occurs in healthy tissue with a few exceptions including wound healing and the changes in endometrial tissue during the menstrual cycle or pregnancy. Thus, neoangiogenesis has become both an important therapeutic as well as diagnostic target.

Many molecular structures that are preferentially or exclusively present in or in the vicinity of growing vascular cells have been described (for a review see, for example, Alessi P, et al. (2004) Biochim. Biophys. Acta. 1654:39-49 and Nanda A and St. Croix B (2004) Curr. Opin. Oncol. 16:44-49) including receptors on the endothelial cells like vascular endothelial growth factor receptor (VEGF-R) and matrix proteins like extra domain B (ED-B) fibronectin. The ED-B domain of fibronectin, a sequence of 91 amino acids identical in mouse, rat and human, which is inserted by alternative splicing into the fibronectin molecule, has been shown to specifically accumulate around neo-vascular structures (Castellani et al. (1994). Int. J. Cancer 59:612-618).

A micrometastasis is a cohesive cluster of malignant cells > 0.2 mm and a cluster of malignant cells < 0.2 mm is called sub-micrometastasis (Van der Westhuizen N. (2002) Laboratory Report; Rampaul RS, et al. (2001) Breast Cancer Res. 3:113-116; Bitterman A., et al. (2002) IMAJ 4:803-809). Micrometastasis which presently can only be detected *in vitro* with a microscope can be angiogenic or non-angiogenic. Most human tumors including primary tumors and metastasis arise without angiogenic activity and exist *in situ* as a microscopic lesion of 0.2 to < 2 mm in diameter for months to years, after which a small percentage may switch to the angiogenic phenotype (Folkman J and Becker K (2000) Acad. Radiol. 7:783-785; Folkman J (2001) Angiogenesis. In Braunwald E, et al., Harrison's Textbook of Internal Medicine, 15th Edition, McGraw-Hill, 517-530). At the cellular level at least four mechanisms of the angiogenic switch have been identified in human and mouse tumors: (1) avascular *in situ* carcinoma can recruit their own blood supply by stimulating neovascularization in an adjacent host vascular bed - the most common process in human tumors, (2) circulating precursor endothelial cells from bone marrow may incorporate into an angiogenic focus, (3) tumors may induce host fibroblast and/or macrophages in the tumor bed to overexpress an angiogenic factor (e.g. , vascular endothelial growth factor (VEGF)); and (4) preexisting vessels can be coopted by tumor cells. The angiogenic switch may also include combinations of these mechanisms (Folkman J (2001) Angiogenesis. In Braunwald E, et al., Harrison's Textbook of Internal Medicine, 15th Edition, McGraw-Hill, 517-530). It is now widely accepted that the "angiogenic switch" is "off" when the effect of pro-angiogenic molecules is balanced by that of anti-angiogenic molecules, and is "on" when the net balance is tipped in favour of angiogenesis. Various signals that trigger this switch have been discovered. Angiogenesis activators are molecular structures as e.g., VEGF family members, VEGFR, NRP-1, Ang1, Thie2, PDGF-BB and receptors, TGF-β1, endoglin, TGF-β receptors, FGF, HGF, MCP-1, Integrins (αᵥβ₃, αᵥβ₅, α₅β₁), VE-cadherin, PECAM (CD31), Ephrins, Plasminogen activators, MMPs, PAI-1, NOS, COX-2, AC133, Chemokins or Id1/Id3. Angiogenesis inhibitors are molecular structures as e.g., VEGFR-1, Ang2, TSP-1,-2, Angiostatin and related plasminogen kringles, Endostatin (collagen XVII fragment), Vasostatin, Platelet factor 4, TIMPs, MMP inhibitors, PEX, Meth-1, Meth-2, IFN-α, -β, -γ, IP-10, IL-4, IL-12, IL-18, Prolactin (M, 16K), VEGI, Fragment of SPARC, Osteopontin fragment or Maspin (Carmeliet P and Jain RK. (2000) Nature 407:249-257; Yancopoulos GD et al. (2000) Nature 407:242-248; Bergers G. and Benjamin LE (2002) Nature Reviews Cancer 3:401-410; Hendrix MJC et al. (2002) Nature Reviews Cancer 3:411-421).

Ntziachristos V, et al. (2000) Proc. Natl. Acad. Sci. U.S.A. 97:2767-2772 describe diffuse optical tomography of fibroadenoma with indocyanine green enhancement. The resolved tumors are primary tumors with a size in excess of 1 cm.

WO 01/23005 A1 describes conjugates of ED-B specific antibodies and various dyes, and their use in the delineation of tumor peripheries. No teaching is provided on the spatial resolution that can be obtained with these conjugates.

McDonald D.M and Choyke P.L (2003) Nat. Med. 9: 713-25 review advances in imaging of angiogenesis. They discuss how magnetic resonance imaging (MRI), computer tomography (CT), positron emission tomography (PET), ultrasonography and optical imaging provide noninvasive methods to obtain images of angiogenesis in animals and humans. They teach that these methods provide their highest resolution on preserved tissue specimen, whereas clinical methods give images of living tissues at much lower resolution and specificity and can not resolve vessels of the microcirculation. It concludes that future challenges include developing new imaging methods that can bridge this resolution gap and specifically identify angiogenic vessels. Presently, no such methods are available.

### Detailed Description of the Invention

Given the difficulties in the prior art to image micrometastasis and newly vascularized or vascularizing structures, i.e. structures, which comprise primarily microvasculature or which are in the process of developing a microvasculature, it has been surprisingly found by the present inventors that such structures can be distinguished by light based diagnosis using conjugates of an angiogenesis specific binding component, in particular ED-B fibronectin specific binding components, and cyanine dye(s). This observation opens the use of near infrared fluorescent imaging to new fields of diagnosis, which require the detection of small diseased structures. Therefore, in a first aspect the present invention provides the use of a conjugate of the general formula (I):

B-(D)ₙ (I),

wherein
- B: stands for an angiogenesis specific binding component,
- D: stands for a cyanine dye, and
- n: is 1 to 5
for the production of a diagnostic for the diagnosis of micrometastasis and small proliferative lesions.

The angiogenesis specific binding component binds to structures, which are preferentially or exclusively present in micrometastasis, in or in the vicinity of newly formed microvessels or which are present prior or during growth of microvascular structures. Such molecular structures are reviewed in, for example, WO 96/01653, Alessi P, et al. (2004) and Nanda A and St Croix B. (2004). As pointed out above cells forming a micrometastasis and similarly cells of small proliferative lesions express both angiogenic and antiangiogenic factors, which as long as the angiogenesis inhibitors counteract the effect of the angiogenic factors leads to a suppression of angiogenesis. Once the effect of the angiogenic factors prevail they lead to initiation of angiogenesis. Thus, both structures, i. e. angiogenesis activators and inhibitors, which are involved in the regulation of angiogenesis can be an angiogenesis specific binding component within the meaning of the present invention. Angiogenesis activators include without limitation molecular structures like, e.g. ED-B fibronectin (ED-BF), endoglin (CD105) (Burrows FJ et al. (1995) Clin. Cancer Res.. 1: 1623-1634), VEGF family members, vascular endothelial growth factor (VEGFR), NRP-1, Ang1, Thie2, PDGF-BB and receptors, TGF-β1, TGF-β receptors, FGF, HGF, MCP-1, Integrins (αᵥβ₃, αᵥβ₅, α₅β₁), VE-cadherin, PECAM (CD31), Ephrins, Plasminogen activators, MMPs, PAI-1, NOS, COX-2, AC133, chemokines or Id1/Id3. Angiogenesis inhibitors include without limitation molecular structures like, e.g. VEGFR-1, Ang2, TSP-1,-2, Angiostatin and related plasminogen kringles, Endostatin (collagen XVII fragment), Vasostatin, Platelet factor 4, TIMPs, MMP inhibitors, PEX, Meth-1, Meth-2, IFN-α, -β, -γ, IP-10, IL-4, IL-12, IL-18, Prolactin (*M*, 16K), VEGI, Fragment of SPARC, Osteopontin fragment or Maspin (Carmeliet P and Jain RK (2000) Nature 407:249-257; Yancopoulos GD et al. (2000) Nature 407:242-248; Bergers G and Benjamin LE (2002) Nature Reviews Cancer 3:401-410; Hendrix MJC et al. (2002) Nature Reviews Cancer 3:411-421). In a preferred embodiment the angiogenesis specific binding component include ED-BF, VEGFR, or endoglin. Out of those ED-BF is a particular preferred target structure. ED-BF is splice variant of fibronectin also called oncofoetal fibronectin, which is specifically formed in newly grown microvascular structures during angiogenesis.

The component that binds to these structures is preferably a peptide (amino acid chain with two to 50 amino acid residues), a protein (amino acid chains with more than 50 amino acid residues), a nucleic acid, a small molecule, or a sugar.

Preferred proteins or peptides are ligands of receptors, which are preferentially or exclusively expressed in micrometastasis and/or nearly vascularized or vascularizing structures, in particular vascular endothelial growth factor (VEGF), and antibodies, including human, humanized and chimeric antibodies; antibody binding domain comprising fragments, e.g. Fv, Fab, Fab', F(ab')₂, Fabc, Facb; single chain antibodies, e.g. single chain Fvs (scFvs); and diabodies.

A large variety of such antibodies has been described in the literature and include for ED-BF L19 and E8 (see Viti F. et al. (1999) Cancer Res. 59:347-352), the BC-1 monoclonal antibody described in EP 0 344 134 B1, which is obtainable from the hybridoma deposited at the European Collection of Animal Cell Cultures, Porton Down, Salisbury, UK under the number 88042101 or a chimeric or humanized version thereof, the antibodies against ED-BF with the specific V_{L} and V_{H} sequences disclosed in WO 97/45544 A1, the antibodies against ED-BF with the specific V_{L} and V_{H} sequences disclosed in WO 99/5857 A2, the antibodies against ED-BF with the specific V_{L} and V_{H} sequences disclosed in WO 01/62800 A1 and AP38 and AP39 (Marty C, et al. (2001) Protein Expr. Purif. 21:156-64). Antibodies specific to ED-BF have been reviewed in Ebbinghaus C, et al. (2004) Curr Pharm Des. 10:1537-49. All these antibodies or antibody binding fragments thereof can be used as angiogenesis specific binding component in a preferred use of the present invention. Particularly preferred antibodies are L19, E8, AP 38 and AP 39 or binding domain comprising fragments thereof.

Antibodies for VEGF-R include Bevacizumab (Avastin^{™}, rhumAb-VEGF developed by Genentech and Roche), the anti-VEGFR-1 antibody mAb 6.12, the fully human anti-VEGFR-2 antibodies IMC-2C6 and IMC-1121, the fully human anti-VEGFR-3 mAb HF4-3C5 (all Imclone Systems Inc.), and KM-2550 (Kyowa Hakko Kogyo Co Ltd), an anti-VEGFR-1 antibody (Salgaller ML (2003) Current Opinion in Molecular Therapeutics 5(6):657-667). Antibodies for endoglin include: SN6h, SN6, SN6a, SN6j, P3D1, P4A4, 44G4, GRE, E-9, CLE-4, RMAC8, PN-E2, MAEND3, TEC4, TEC11, A11, 8E11. Clone SN6h has been used extensively to study expression of endoglin in different tumor entities by immunohistochemistry (Wikström P. et al. (2002) The Prostate 51:268-275; Li C. et al. (2003) Br. J. Cancer 88:1424-1431; Saad R.S. et al. (2004) Modem Pathol. 17: 197-203). Of the same SN6 series antibodies SN6, SN6a and SN6j have been described (She X. et al. (2004) Int. J. Cancer 108:251-257). For the antibody clones P3D1, P4A4, 44G4, GRE, E-9, CLE-4, RMAC8, PN-E2, MAEND3, TEC4, TEC11 the binding epitopes of endoglin have been determined (Pichuantes S. et al. (1997) Tissue antigens 50:265-276). For some of these antibodies and antibody clone A11 the differential expression of endoglin has been investigated on normal and tumor tissues of human origin (Duff S. E. et al. (2003) FASEB J. 17:984-992). WO 02/02614 discloses further endoglin specific antibodies, e.g. scFv C4. In one of the last publications on antibodies against CD 105 the clone 8E11 was investigated for its prediction of metastatic risk in breast cancer patients by immunohistochemistry (Dales J.P. et al. (2004) Br. J. Cancer 90:1216-1221). All these antibodies or antibody binding fragments thereof can be used as angiogenesis specific binding component in a preferred use of the present invention.

It is well known in the art that nucleic acids can possess specific binding properties, thus, the angiogenesis specific binding component can also be a nucleic acid. Preferably, such nucleic acids include DNA, RNA, aptamers, and PNA, wherein aptamers are particularly preferred. Methods to identify specifically binding aptamers are well known in the art and are described, for example, in WO 93/24508 A1, WO 94/08050 A1, WO 95/07364 A1, WO 96/27605 A1, and WO 96/34875 A1. The methods disclosed in these documents are hereby specifically referenced and can be used in the identification of angiogenesis specific binding aptamers useable in the present invention. Preferred aptamers employed in the use of the present invention specifically recognize ED-BF, endoglin or VEGFR.

With the advent of high throughput screening of small molecules, i.e. non peptidly, non nucleic acid compounds, of a molecular weight lower than 1.000 g/mol, preferably lower than 500 g/mol, it has been possible to identify small molecules with particular binding properties. Such small molecules can equally be employed as one component of the conjugate usable according to the present invention. A preferred small molecule is 2,2-diphenylethylamine, which has been identified to specifically bind to ED-BF (Scheuermann J. (2002) Isolation of binding molecules to the EDB domain of fibronectin, a marker of angiogenesis. Dissertation submitted to Swiss Federal Inst. of Technology, Zurich).

In a preferred use of the present invention the cyanine dye is selected from the group consisting of carbocyanine, dicarbocyanine, and tricarbocyanine. The synthesis of cyanine dyes useable according to the present invention can be carried out using the methods known in the state of the art and which are exemplified in, e.g. Hamer F.M. The Cyanine Dyes and Related Compounds, John Wiley and Sons, New York 1964; Ernst LA, et al. (1989) Cytometry 10:3-10; Southwick PL, et al., (1990) Cytometry 11:418-430; Lansdorp PM et al., (1991) Cytometry 12:723-730; Mujumdor RB et al., (1993) Bioconjugate Chem. 4:105-11; Mujumdor SR et al., (1996) Bioconjugate Chem. 7:356-62; Flanagan JH et al., (1997) Bioconjugate Chem. 8:751-56; Keil D et al., (1991) Dyes and Pigments 17:19-27; Terpetschnig E and Lakowicz JR (1993) Dyes and Pigments 21:227-34; Terpetschnig E et al., (1994) Anal. Biochem. 217: 197-204; Lindsey JS et al., (1989) Tetrahedron 45:4845-66; Górecki T et al., (1996) J. Heterocycl. Chem. 33, 1871-6; Narayanan N and Patonay G (1995) J. Org. Chem. 60:2391-5, 1995; and Terpetschnig E et al., (1993) J. Fluoresc. 3:153-155. Additional processes are described in patent publications US 4,981,977; US 5,688,966; US 5,808,044; EP 0 591 820 A1; WO 97/42976; WO 97/42978; WO 98/22146; WO 98/26077; and EP 0 800 831.

Moreover, indotricarbocyanines with altered substituents were synthesized and coupled to biomolecules (described in, e.g. Becker A et al., Photochem. Photobiol. 72, 234, 2000; Licha K et al., Bioconjugate Chem. 12, 44, 2001; Becker A et al., Nature Biotechnol. 19, 327, 2001; Bugaj JE et al., J. Biomed. Optics 6, 122, 2001;Achilefu S et al., J. Med. Chem. 45, 2003, 2002). Other examples are found in particular in the publications WO 00/61194 ("Short-Chain Peptide Dye Conjugates as Contrast Agents for Optical Diagnostics"), WO 00/71162, WO 01/52746, WO 01/52743 and WO 01/62156. Another process for the production of an indotricarbocyanine dye is a simple access via 4-substituted pyridines. Various 4-substituted pyridines can be converted by means of the Zincke reaction (Zincke-König reaction, see Römpps Chemie Lexikon [Römpps Chemical Dictionary], 10th Edition, page 5067) in high yields into meso-substituted glutaconaldehyde-dianilide, which are precursors to cyanine dyes.

In a particular preferred embodiment of the present invention the cyanine dye has the general formula (II) 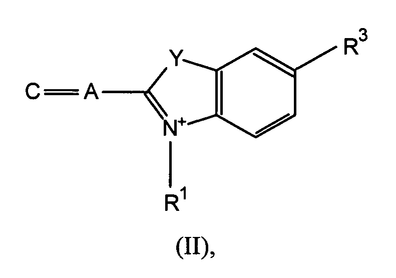 wherein C stands for a radical (III) or (IV) 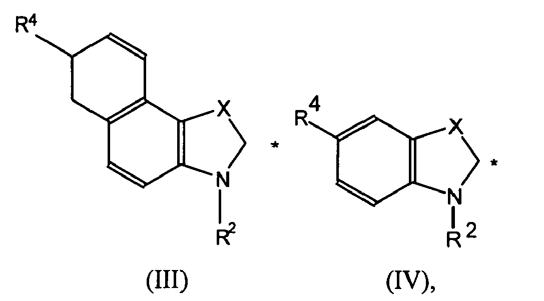 wherein the position that is labeled with the star means the point of linkage with radical A and can stand for the group (V), (VI), (VII), (VIII) or (IX) 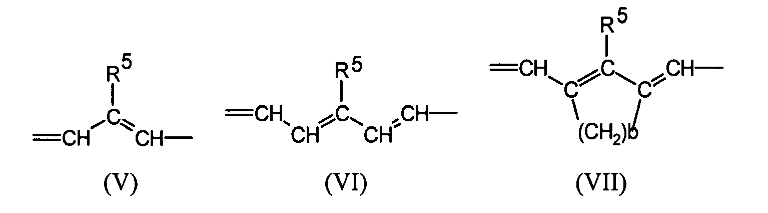 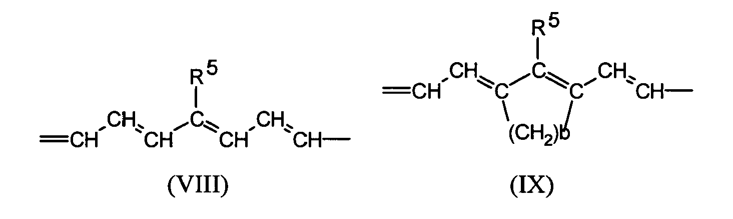 wherein
R¹ and R², independently of one another, stand for a C₁-C₄-sulfoalkyl chain, e.g. sulfomethyl, sulfoethyl, *n*-sulfopropyl, *iso*-sulfopropyl, sulfobutyl, *iso*-sulfobutyl, *sec-*sulfobutyl, *tert*-isobutyl; or a saturated or unsaturated, branched or straight-chain C₁-C₅₀-alkyl chain, e.g. CH₃, C₂H₅, C₃H₇, C₄H₉, C₅H₁₁, C₆H₁₃, C₇H₁₅, C₈H₁₇, C₉H₁₅, C₁₀H₂₁, C₁₁H₂₃, C₁₂H₂₃, C₁₃H₂₇, C₁₄H₁₉, C₁₅H₃₁, C₁₆H₃₃, C₁₇H₃₅, C₁₈H₃₇, C₁₉H₃₉, C₂₀H₄₁, C₂₁H₄₃, C₂₂H₄₅, C₂₃H₄₇, C₂₄H₄₉, C₂₅H₅₁, C₂₆H₅₃, C₂₇H₅₅, C₂₈H₅₇, C₂₉H₅₉, C₃₀H₆₁, C₃₁H₆₃, which optionally is substituted by 0 to 15, e.g. 1, 2, 3, 4, 5, 6, 7, 8, 9, 10, 11, 12, 13, 14, or 15, oxygen atoms and/or by 0 to 3 carbonyl groups, e.g. 1, 2, or 3, and/or with 0 to 5, e.g. 1, 2, 3, 4, 5, hydroxyl groups or is optionally interrupted by 0 to 15, e.g. 1, 2, 3, 4, 5, 6, 7, 8, 9, 10, 11, 12, 13, 14, or 15, oxygen atoms and/or by 0 to 3. e.g. 1, 2, or 3, carbonyl groups and/or can be substituted with 0 to 5, e.g. 1, 2, 3, 4, or 5, hydroxyl groups;
R³ stands for B or a linker connected to B, wherein the linker is a branched or straight-chain carbohydrate chain with up to 20 carbon residues, in particular methyl, ethyl, propyl, *iso*-propyl, butyl, *iso*-butyl, *tert*-butyl, pentyl, hexyl, pentyl, otyl, nonyl, decyl, undecyl, dodecyl, tridecyl, tetradecyl, pentadecyl, hexadecyl, heptadecyl, octadecyl, nonadecyl, eicosyl, which is substituted with one or more -OH, -COOH, -SO₃ groups and/or optionally interrupted one or more times (preferably 2, 3, 4, 5 or 6 times) by -O-, -S-, -CO-, - CS-, -CONH, -NHCO-, NHCSNH-, -SO₂-, -PO₄⁻-, -aryl- and/or -NH- group;
R⁴ stands for the group -COOE¹, -CONE¹E², -NHCOE¹, -NHCONHE¹, -NE¹E², - OE¹, -OSO₃E¹, -SO₃E¹, -SO₂NHE¹ or-E¹, wherein
E¹ and E², independently of one another, stand for a hydrogen atom, a C₁-C₄-sulfoalkyl chain, e.g. sulfomethyl, sulfoethyl, *n*-sulfopropyl, *iso*-sulfopropyl, sulfobutyl, *iso*-sulfobutyl, *sec-*sulfobutyl, *tert*-isobutyl; a saturated or unsaturated, branched or straight-chain C₁-C₅₀-alkyl chain, e.g. CH₃, C₂H₅, C₃H₇, C₄H₉, C₅H₁₁, C₆H₁₃, C₇H₁₅. C₈H₁₇, C₉H₁₉, C₁₀H₂₁, C₁₁H₂₃, C₁₂H₂₃, C₁₃H₂₇, C₁₄H₁₉, C₁₅H₃₁, C₁₆H₃₃, C₁₇H₃₅, C₁₈H₃₇, C₁₉H₃₉, C₂₀H₄₁, C₂₁H₄₃, C₂₂H₄₅, C₂₃H₄₇, C₂₄H₄₉, C₂₅H₅₁, C₂₆H₅₃, C₂₇H₅₅, C₂₈H₅₇, C₂₉H₅₉, C₃₀H₆₁, C₃₁H₆₃, which optionally is interrupted by 0 to 15 oxygen atoms, e.g. 1, 2, 3, 4, 5, 6, 7, 8, 9, 10, 11, 12, 13, 14, or 15, and/or by 0 to 3 carbonyl groups, e.g. 1, 2, or 3, and/or is substituted with 0 to 5 hydroxyl groups, e.g. 1, 2, 3, 4, or 5;
R⁵ stands for a hydrogen atom, or a fluorine, chlorine, bromine or iodine atom, methyl, ethyl, propyl or iso-propyl;
b means the number 2 or 3; and
X and Y, independently of one another, stand for O, S, =C(CH₃)₂ or-(CH=CH)-,
as well as pharmaceutically acceptable salts and solvates of these compounds.

In a further preferred embodiment (i) the cyanine dye usable according to the present invention has the general formula (X) 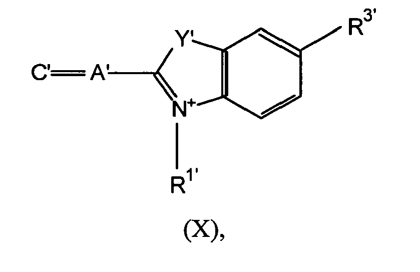 wherein C' stands for a radical (XI) or (XII) 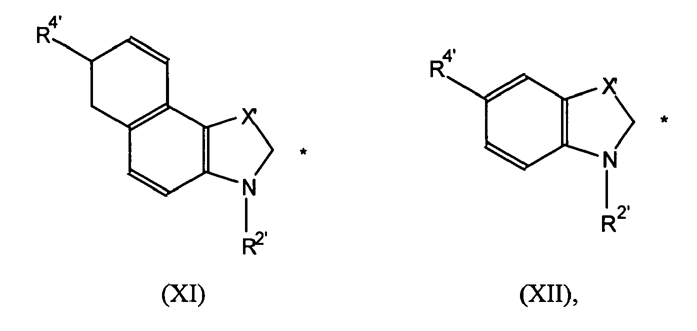 wherein the position that is labeled with the star means the point of linkage with radical A' and can stand for the group (XIII), (XIV), (XV), (XVI) or (XVII) 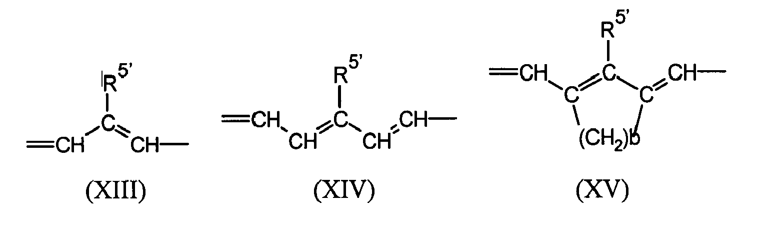 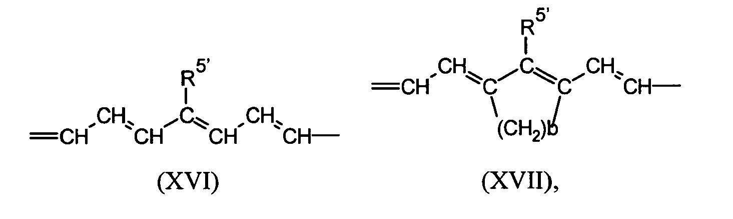 wherein radical (XV) or (XVII) optionally can be substituted with a C₁ to C₄-alkyl radical, e.g. methyl, ethyl, propyl, *iso*-propyl, butyl, *iso*-butyl, *sec*-butyl, *tert-butyl,*
wherein
R^{1'} stands for a C₁-C₄-sulfoalkyl chain, e.g. sulfomethyl, sulfoethyl, n-sulfopropyl, *iso*-sulfopropyl, sulfobutyl, *iso*-sulfobutyl, *sec*-sulfobutyl, *tert*-isobutyl; a saturated or unsaturated, branched or straight-chain C₁-C₅₀-alkyl chain, e.g. CH₃, C₂H₅, C₃H₇, C₄H₉, C₅H₁₁, C₆H₁₃. C₇H₁₅, C₈H₁₇, C₉H₁₉, C₁₀H₂₁, C₁₁H₂₃, C₁₂H₂₃, C₁₃H₂₇, C₁₄H₁₉, C₁₅H₃₁, C₁₆H₃₃, C₁₇H₃₅, C₁₈H₃₇, C₁₉H₃₉, C₂₀H₄₁, C₂₁H₄₃, C₂₂H₄₅, C₂₃H₄₇, C₂₄H₄₉, C₂₅H₅₁, C₂₆H₅₃, C₂₇H₅₅, C₂₈H₅₇, C₂₉H₅₉, C₃₀H₆₁, C₃₁H₆₃, which optionally is substituted by 0 to 15, e.g. 1, 2, 3, 4, 5, 6, 7, 8, 9, 10, 11, 12, 13, 14, or 15, oxygen atoms and/or by 0 to 3 carbonyl groups, e.g. 1, 2, or 3, and/or can be substituted with 0 to 5, e.g. 1, 2, 3, 4, 5, hydroxyl groups or is optionally interrupted by 0 to 15, e.g. 1, 2, 3, 4, 5, 6, 7, 8, 9, 10, 11, 12, 13, 14, or 15, oxygen atoms and/or by 0 to 3. e.g. 1, 2, or 3, carbonyl groups and/or can be substituted with 0 to 5, e.g. 1, 2, 3, 4, or 5, hydroxyl groups; or M'-R^{6'};
R^{2'} stands for a C₁-C₄-sulfoalkyl chain, a C₁-C₄-sulfoalkyl chain, e.g. sulfomethyl, sulfoethyl, *iso*-sulfopropyl, sulfobutyl, *iso*-sulfobutyl, *sec*-sulfobutyl, *tert*-isobutyl, a saturated or unsaturated, branched or straight-chain C₁-C₅₀-alkyl chain, e.g. CH₃, C₂H₅, C₃H₇, C₄H₉, C₅H₁₁, C₆H₁₃, C₇H₁₅, C₈H₁₇, C₉H₁₉, C₁₀H₂₁, C₁₁H₂₃, C₁₂H₂₃, C₁₃H₂₇, C₁₄H₁₉, C₁₅H₃₁, C₁₆H₃₃, C₁₇H₃₅, C₁₈H₃₇, C₁₉H₃₉, C₂₀H₄₁, C₂₁H₄₃, C₂₂H₄₅, C₂₃H₄₇, C₂₄H₄₉, C₂₅H₅₁, C₂₆H₅₃, C₂₇H₅₅, C₂₈H₅₇, C₂₉H₅₉, C₃₀H₆₁, C₃₁H₆₃, which optionally is substituted by 0 to 15, e.g. 1, 2, 3, 4, 5, 6, 7, 8, 9, 10, 11, 12, 13, 14, or 15, oxygen atoms and/or by 0 to 3 carbonyl groups, e.g. 1, 2, or 3, and/or can be substituted with 0 to 5, e.g. 1, 2, 3, 4, 5, hydroxyl groups or is optionally interrupted by 0 to 15, e.g. 1, 2, 3, 4, 5, 6, 7, 8, 9, 10, 11, 12, 13, 14, or 15, oxygen atoms and/or by 0 to 3. e.g. 1, 2, or 3, carbonyl groups and/or can be substituted with 0 to 5, e.g. 1, 2, 3, 4, or 5, hydroxyl groups; or M'-R^{7'};
R^{3'}, R^{4'}, R^{6'} and R^{7'}, independently of one another, stand for the group -COOE^{1'}, - CONE^{1'}E^{2'} -NHCOE^{1'} , -NHCONHE^{1'}, -NE^{1'}E^{2'}, -OE^{1'}, -OSO₃E^{1'}, -SO₃E^{1'}, -SO₂NHE^{1'} or - E^{1'}, wherein
E^{1'} and E^{2'}, independently of one another, stand for a hydrogen atom, a C₁-C₄-sulfoalkyl chain, e.g. sulfomethyl, sulfoethyl, *iso*-sulfopropyl, sulfobutyl, *iso*-sulfobutyl, *sec*-sulfobutyl, *tert*-isobutyl, a saturated or unsaturated, branched or straight-chain C₁-C₅₀-alkyl chain, e.g. CH₃, C₂H₅, C₃H₇, C₄H₉, C₅H₁₁, C₆H₁₃, C₇H₁₅, C₈H₁₇, C₉H₁₉, C₁₀H₂₁, C₁₁H₂₃, C₁₂H₂₃, C₁₃H₂₇, C₁₄H₁₉, C₁₅H₃₁, C₁₆H₃₃, C₁₇H₃₅, C₁₈H₃₇, C₁₉H₃₉, C₂₀H₄₁, C₂₁H₄₃, C₂₂H₄₅, C₂₃H₄₇, C₂₄H₄₉, C₂₅H₅₁, C₂₆H₅₃, C₂₇H₅₅, C₂₈H₅₇, C₂₉H₅₉, C₃₀H₆₁, C₃₁H₆₃, which optionally is interrupted by 0 to 15 oxygen atoms, e.g. 1, 2, 3, 4, 5, 6, 7, 8, 9, 10, 11, 12, 13, 14, 15 and/or by 0 to 3 carbonyl groups, e.g. 1, 2, 3, and/or is substituted with 0 to 5 hydroxyl groups, e. g. 1, 2, 3, 4, 5;
M' stands for CH₂-CH₂ or CH₂-CH₂-CH₂;
R^{5'} stands for -Q'-CH₂-R^{8'};
Q' stands for C₁ to C₅ alkyl, e.g. methyl, ethyl, *n*-propyl, *iso*-propyl, butyl, *iso-*butyl, *sec*-butyl, *tert*-butyl, *n*-pentyl, 1-methylbutyl, 2-methylbutyl, 3-methylbutyl, 1-dimethylpropyl, neopentyl, whereby the C atoms are optionally substituted by O or S; or stands for 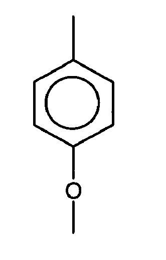 R^{8'} stands for -CO-NH-R^{9'}-R^{10'}, -NH-CS-NH- R^{9'}-R^{10'} or -NH-CO- R^{9'}-R^{10'},
wherein
R^{9'} is selected from the group consisting of unbranched C₂-C₁₃ alkyl, e.g. ethyl, propyl, butyl pentyl, hexyl hepty octyl, nonyl, decyl, undecyl, dodecyl and tridecyl, in which one or more C atoms, e.g. 1, 2, 3, 4, are optionally replaced by O or S, and
R^{10'} is B or the residual part of a coupling moiety, which is linked to B, and
b' means the number 2 or 3; and
X' and Y', independently of one another, stand for O, S, =C(CH₃)₂ , =C(C₂H₅)₂, =C(C₃H₇)₂, =C(*iso*C₃H₇)₂, =C(C₄H₉)₂, or -(CH=CH)-,
as well as pharmaceutically acceptable salts and solvates of these compounds.

In a more preferred embodiment (ii) of the cyanine dyes usable according to the present invention R^{5'}, R^{8'}, R^{9'}, R^{10'}, E^{1'}, E^{2'}, M' and Q' have the meaning as outlined above for embodiment (i) and
A' stands for a radical (XVI) or (XVII), wherein radical (XVII) optionally can be substituted in *para*-position with a C₁ to C₄-alkyl radical, e.g.methyl, ethyl, propyl, *iso-*propyl, *iso*-butyl, *sec*-butyl, or *tert*-butyl;
C' stands for a radical (XII);
R^{1'} stands for M-R^{6'};
R^{2'} stands for M-R^{7'};
R^{3'}, R^{4'}, R^{6'} and R^{7'}, independently of one another, stand for SO₃H or H, with the proviso that at least three of R^{3'}, R^{4'}, R^{6'} and R^{7'} are SO₃H, and
X' and Y', independently of one another, stand for O, S, =C(CH₃)₂ , =C(C₂H₅)₂, =C(C₃H₇)₂, =C(*iso*C₃H₇)₂, or =C(C₄H₉)₂,
b' is 3.

In a more preferred embodiment (iii) of the cyanine dyes usable according to the present invention C' R^{1'}, R^{2'}, R^{3'}, R^{4'}, R^{5'}, R^{6'}, R^{7'}, R^{8'}, R^{9'}, R^{10'} , E^{1'}, E^{2'}, X', Y' and b' have the meaning as outlined above for embodiment (i); or R^{5'}, R^{8'}, R^{9'}, R^{10'}, E^{1'} and E^{2'} have the meaning as outlined above for embodiment (i) and C', R^{1'}, R^{2'}, R^{3'}, R^{4'}, R^{6'}, R^{7'}, X', Y' and b' have the meaning as outlined above for embodiment (ii) and
A' stands for the radical with the formula (XVI);
M' stands for CH₂-CH₂; and
Q' stands for C₁ to C₅ alkyl, whereby the C atoms are optionally substituted by O or S.

In a more preferred embodiment (iv) of the cyanine dyes usable according to the present invention A', C', R^{1'} R^{2'}, R^{3'}, R^{4'}, R^{5'}, R^{6'}, R^{7'}, R^{8'}, R^{9'} R^{10'}, E^{1'} E^{2'} M', X', Y' and b' have the meaning as outlined above for embodiment (i); R^{5'}, R^{8'}, R^{9'}, R^{10'}, E^{1'}, E^{2'} and M' have the meaning as outlined above for embodiment (i) and A', C', R^{1'}, R^{2'}, R^{3'}, R^{4'}, R^{6'}, R^{7'} X', Y' and b' have the meaning as outlined above for embodiment (ii); C' R^{1'} R^{2'}, R^{3'}, R^{4'}, R^{5'}, R^{6'} R^{7'} R^{8'}, R^{9'}, R^{10'}, E^{1'}, E^{2'}, X', Y' and b' have the meaning as outlined above for embodiment (i) and A' and M' have the meaning as outlined above for embodiment (iii); or R^{5'}, R^{8'}, R^{9'}, R^{10'}, E^{1'} and E^{2'} have the meaning as outlined above for embodiment (i), C', R^{1'}, R^{2'}, R^{3'}, R^{4'}, R^{6'} R^{7'}, X', Y' and b' have the meaning as outlined above for embodiment (ii) and A' and M' have the meaning as outlined above for embodiment (iii) and
Q' stands for C₁-C₅ alkyl, e.g. methyl, ethyl, propyl, *iso-*propyl, butyl, *iso-*butyl, *sec*-butyl, *tert*-butyl, n-pentyl, 1-methylbutyl, 2-methylbutyl, 3-methylbutyl, 1-dimethylpropyl, neopentyl.

In a more preferred embodiment (v) of the cyanine dyes usable according to the present invention C' R^{1'} R^{2'}, R^{3'}, R^{4'}, R^{5'}, R^{6'}, R^{7'} , R^{8'}, R^{9'}, R^{10'}, E^{1'}, E^{2'}, M', X', Y' have the meaning as outlined above for embodiment (i); and R^{5'}, R^{8'}, R^{9'}, R^{10'}, E^{1'}, E^{2'} and M' have the meaning as outlined above for embodiment (i) and C', R^{1'}, R^{2'}, R^{3'}, R^{4'} R^{6'} R^{7'}, X', and Y' have the meaning as outlined above for embodiment (ii) and
A' stands for the radical with the formula (XVII)
b' means 3, and
Q' stands for

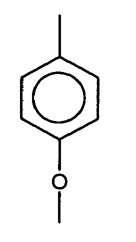

In a more preferred embodiment (vi) of the cyanine dyes usable according to the present invention A', C', R^{1'}, R^{2'} R^{3'}, R^{4'} R^{5'}, R^{6'}, R^{7'}, R^{9'}, R^{10'}, E^{1'}, E^{2'} M', Q', X', Y' have the meaning as outlined above for embodiment (i); R^{5'}, R^{9'}, R^{10'}, E^{1'}, E^{2'}, M' and Q' have the meaning as outlined above for embodiment (i) and A', C', R^{1'}, R^{2'}, R^{3'}, R^{4'}, R^{6'}, R^{7'}, X', Y' and b have the meaning as outlined above for embodiment (ii); C' R^{1'}, R^{2'}, R^{3'}, R^{4'}, R^{5'}, R^{6'} R^{7'}, R^{9'}, R^{10'}, E^{1'}, E^{2'}, X', Y' and b' have the meaning as outlined above for embodiment (i) and A', M' and Q' have the meaning as outlined above for embodiment (iii); or R^{5'}, R^{9'}, R^{10'}, E^{1'} and E^{2'} have the meaning as outlined above for embodiment (i), C', R^{1'}, R^{2'}, R^{3'}, R^{4'} R^{6'} R^{7'} X', Y' and b' have the meaning as outlined above for embodiment (ii) and A', M', Q' and have the meaning as outlined above for embodiment (iii) and
R^{8'} stands for CO-B or NH-B.

Especially preferred indotricarbocyanine dyes, which can be used according to the invention are selected from the dyes with formulas (XVIII) to (XXXVI) that are listed in Table 1 below show the structure of the dyes prior to coupling to B and comprise either a maleinimide (maleimide) or bromoacetyl coupling moiety, which facilitates coupling to thiol-group containing angiogenesis specific binding components. In the resulting conjugates the respective coupling moiety will in some embodiments not be present anymore, if it was a leaving group, or only parts of it will remain in the conjugate called residual part of a coupling moiety. It will be apparent to someone of skill in the art that other moieties instead of the maleinimide (maleimide) or bromoacetyl coupling moieties depicted below can be substitued in below structures, including, for example, chloroacetyl, iodoacetyl, chloroacetamido, bromoacetamido, iodoacetamido, chloroalkyl, bromoalkyl, iodoalkyl, pyridyl disulfide and vinyl sulfonamide, to effect coupling reactions to B.

**Table 1**

| Preferred dyes which can be used for coupling to an angiogenesis specific binding component: | |
|---|---|
| **Formula** | |
| (XVIII) Example 1 | 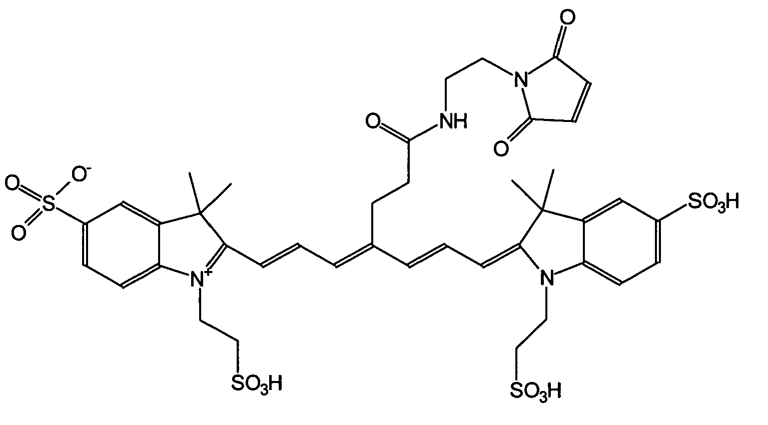 |
| (XIX) Example 2 | 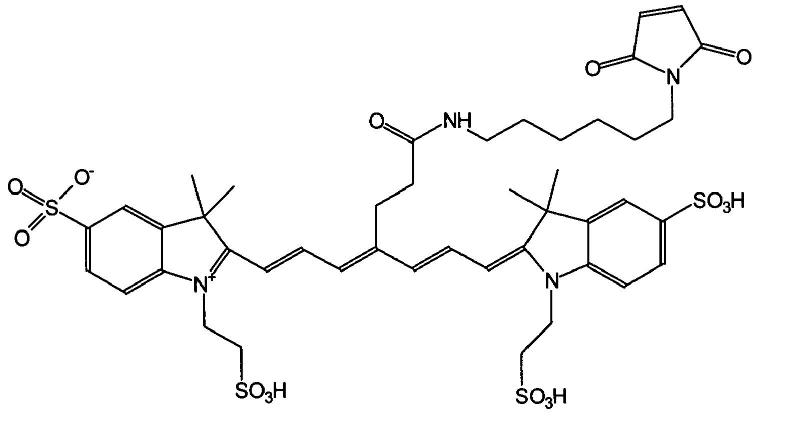 |
| (XX) Example 3 | 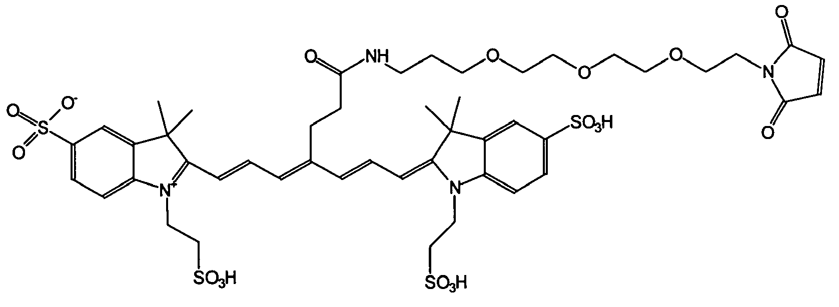 |
| (XXI) Example 4 | 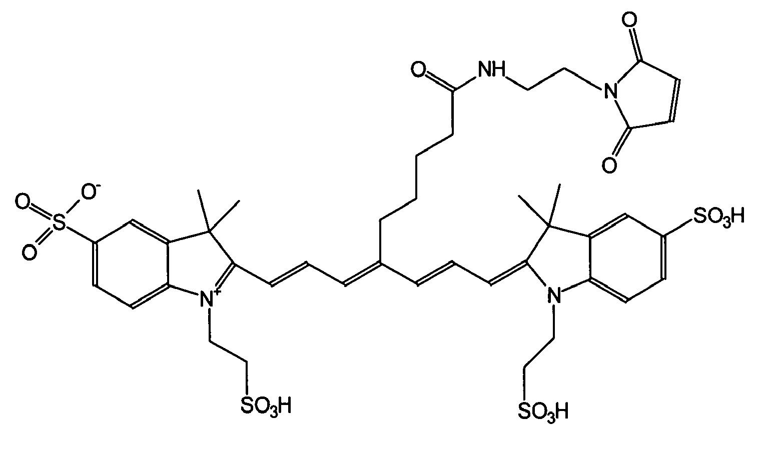 |
| (XXII) Example 5 | 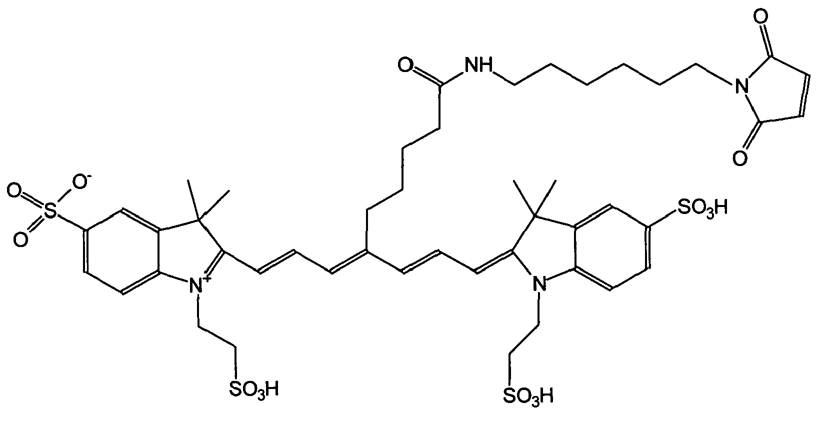 |
| (XXIII) Example 6 | 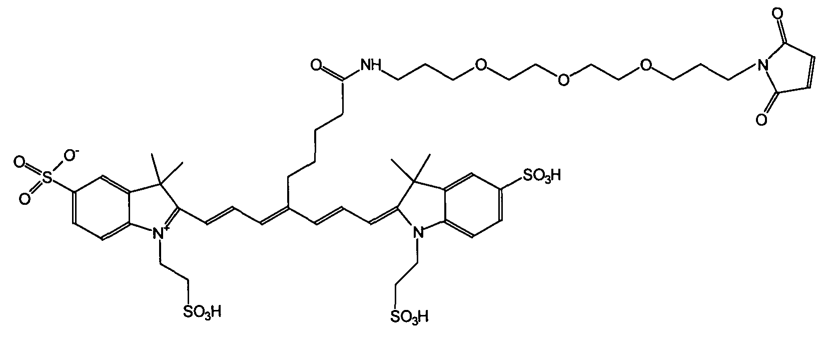 |
| (XXIV) Example 7 | 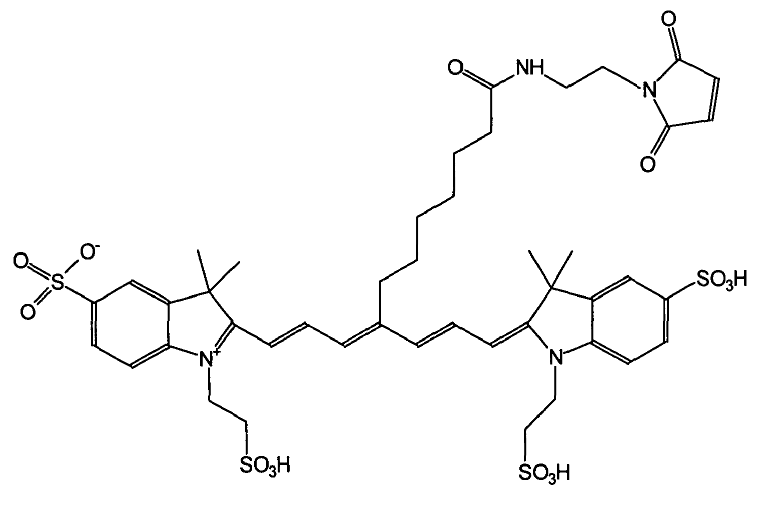 |
| (XXV) Example 8 | 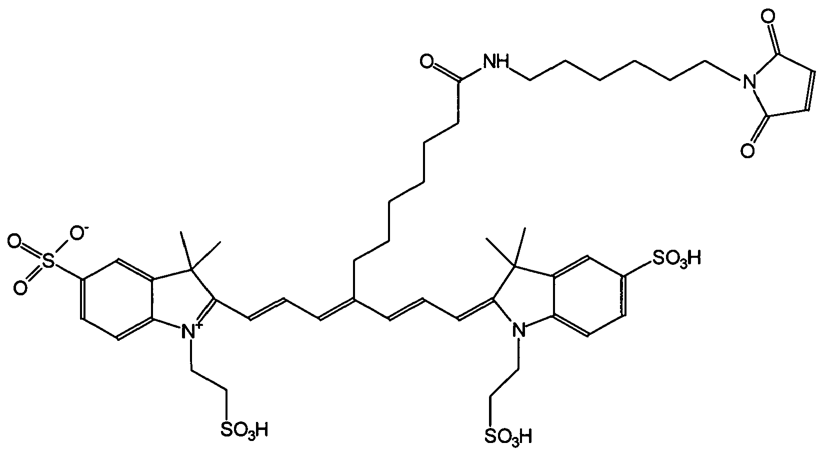 |
| (XXVI) Example 9 | 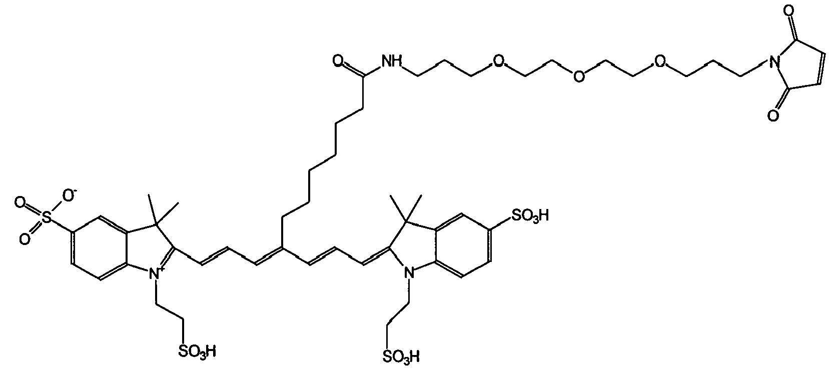 |
| (XXVII) Example 10 | 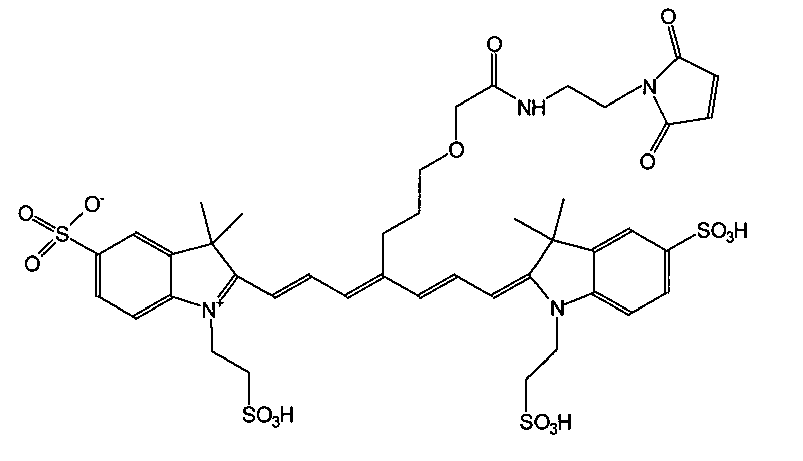 |
| (XXVIII) Example 11 | 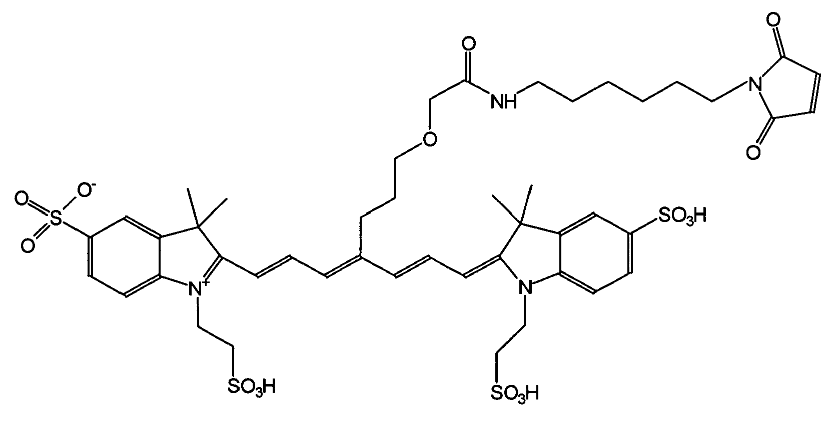 |
| (XXIX) Example 12 | 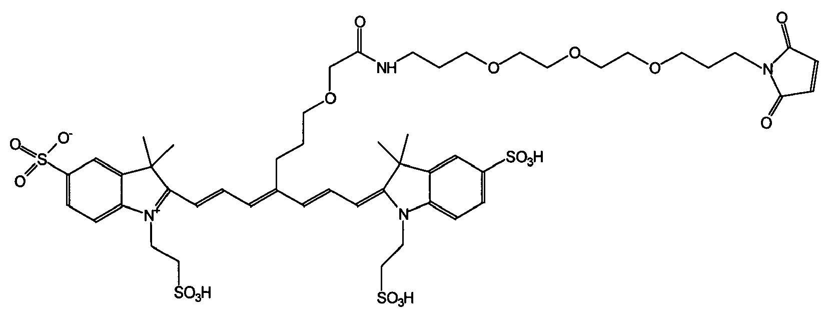 |
| (XXX) Example 13 | 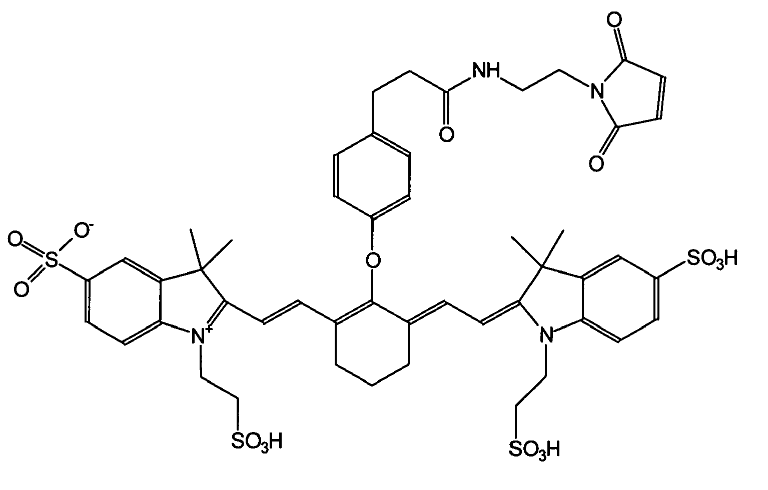 |
| (XXXI) Example 14 | 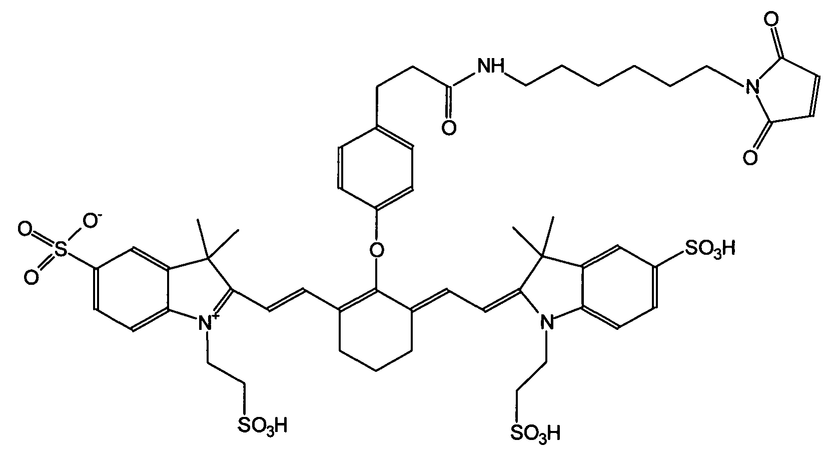 |
| (XXXII) Example 15 | 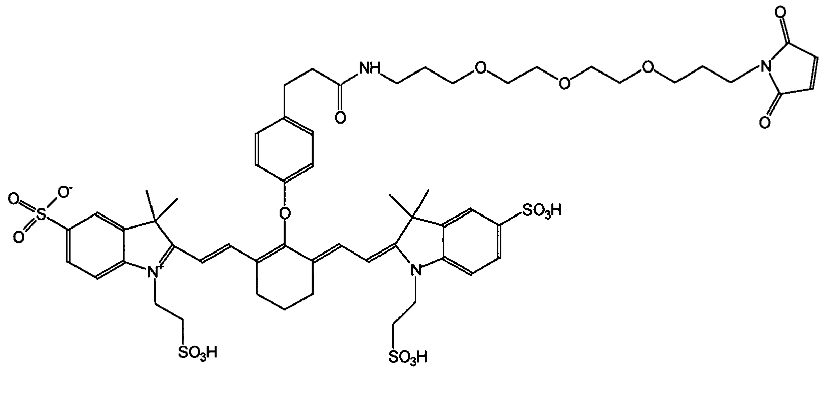 |
| (XXXIII) Example 16 | 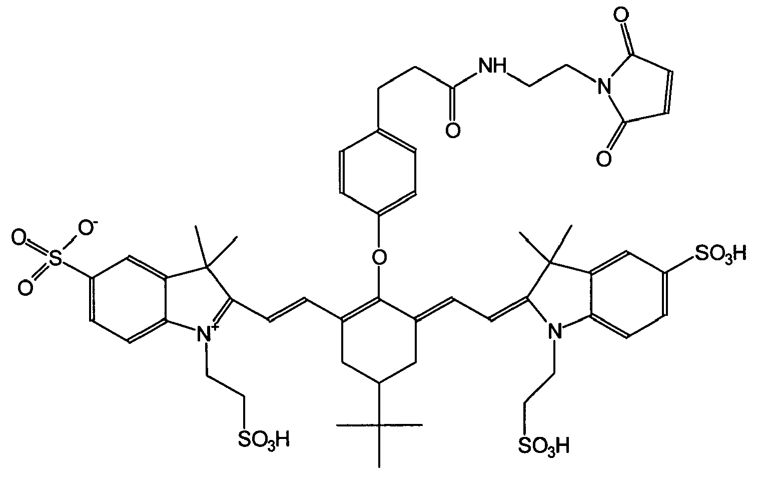 |
| (XXXIV) Example 18 | 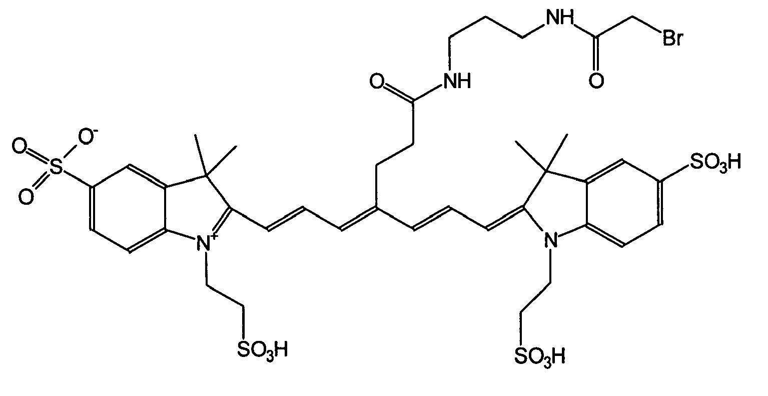 |
| (XXXV) Example 17 | 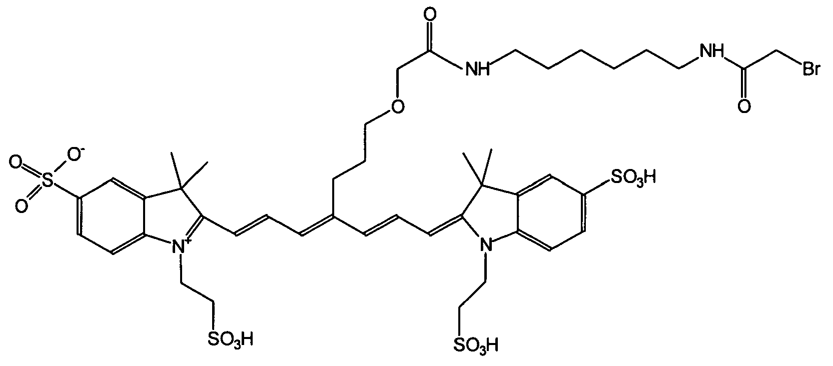 |
| (XXXVI) Example 19 | 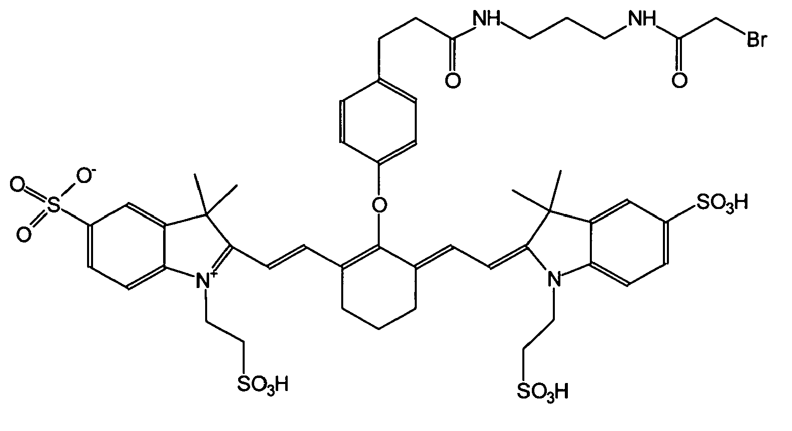 |

The cyanine dyes coupled to B via R^{5'} in the middle of the cyanine dye show a particular good quantum yield and a surprisingly low or even no reduction of the quantum yield once coupled to an angiogenesis specific binding component. Therefore, the use of the cyanine dyes according to embodiments (i) to (vi) and the specific cyanine dyes according to structures (XVIII) to (XXXVI) is in the context of the present invention particularly preferred.

The appropriate method for coupling the respective cyanine dye and the respective angiogenesis specific binding component primarily depends on the chemical nature of the angiogenesis specific binding component. A large variety of residues or groups are known in the art, which are naturally present or can be introduced into the various angiogenesis specific binding components, e.g. -NH₂, -COOH, -SH, -OH etc.. These groups can then form covalent bonds with groups attached to the cyanine dyes, which show a good reactivity, towards the other group resulting in the coupling of the two components. Of course it is also possible to inverse the order, i.e. attach the reactable group to the cyanine dye and the reactive group to the angiogenesis specific binding component. Based on this teaching the skilled person is able to choose appropriate reactive and reactable groups for each respective pair of a cyanine dye and an angiogenesis specific binding component.

Many proteins or peptides comprise thiol-groups, e.g. of cysteine residues, or can be modified to comprise thiol groups. Therefore, if the conjugate used in the present invention comprises a peptide or protein and (a) cyanine dye(s) it is preferred that the cyanine dyes mentioned above comprise prior to coupling to the protein or peptide a thiol group-reactive coupling moiety. Thiol group-reactive functionalities are well known in the art and comprise, e.g. maleinimide (maleimide), chloroacetyl, bromoacetyl, iodoacetyl, chloroacetamido, bromoacetamido, iodoacetamido, chloroalkyl, bromoalkyl, iodoalkyl, pyridyl disulfide and vinyl sulfonamide. Thus, in a preferred embodiment R³ or R^{10'} in above embodiments represented such a coupling moiety prior to linkage to B. If R³ is a linker connected to B the linker comprises such coupling moiety prior to coupling.

For some coupling moieties, which are not entirely replaced during the coupling reaction, e.g. which are not a leaving group in the coupling reaction, a part of the coupling moiety may remain attached to R³, to the linker or to R^{10'}. This part, which may remain at the junction of the cyanine dye and the angiogenesis specific binding component is referred to as "residual part of the coupling moiety".

The pharmaceutically acceptable salt may be any as long as it forms a non-toxic salt with the cyanine compounds outlined above. Examples include alkali metal salts such as sodium salts, potassium salts; salts of alkaline earth metals such as magnesium salts, calcium salts and the like organic ammonium salts such as triethyl ammonium salts, tributyl ammonium salts, pyridinium salts and the like, salts of amino acids such as lysine, arginine and the like. Preferred salts are sodium salts.

Small proliferative lesions are in a preferred embodiment a primary tumor, a precancerosis, a dysplasia, a metaplasia, psoriasis, psoriatic arthritis, rheumatoid arthritis, endometriosis and/or ocular diseases associated with angiogenesis. In a preferred use of the present invention the conjugate(s) is (are) used for *in vivo* diagnosis of the above indicated diseases and/or micrometastasis.

The use of the present invention can be for routine diagnosis, i.e. for screening for the respectively indicated diseases. However, in a preferred embodiment the conjugates are used once a disease has been diagnosed with, for example, a standard x-ray procedure, e.g. mammography, a whole body scans or MRI. The patient is then examined for further micrometastasis and/or small (additional) primary tumor(s). Such an examination can occur for a better assessment of the severity, e.g. stage of a disease, in order to determine the best treatment options and/or prior, during and/or after a treatment procedure (e.g., drugs, radiation or surgery). If performed prior to a treatment procedure the use of the diagnostic of the present invention allows the determination whether, e.g. micrometastases have already formed in the vicinity of the primary tumor and, thus, whether a lumpectomy or rather a mastectomy is indicated as an example in breast cancer. After treatment the use of the diagnostic of the present invention allows to assess the success of the treatment procedure and to determine subsequent treatment regimens, e.g. radiation or chemotherapy. When used during a surgical procedure it is, for example, possible to detect micrometastasis in tissue, e.g. lymph nodes, surrounding the primary tumor. In this embodiment the use of the present invention allows a more complete removal of tumors or micrometastasis during the procedure.

The use according to the present invention allows the detection of events preceding the onset of angiogenesis, the onset of angiogenesis or angiogenesis, i.e. already formed microvasculature, even when it occurs in very small tissue structures. In a preferred embodiment of the present invention the micrometastasis and/or the small proliferative lesions, in particular the micrometastasis, the precancerosis, the dysplasia, the metaplasia, endometriosis and/or the primary tumor(s), which are detected with the present invention has (have) a diameter of less than 10 mm, preferably of less than 8 mm, more preferably of less than 6 mm, more preferably of less than 5 mm, more preferably of less than 4 mm, more preferably of less than 3 mm, more preferably of less than 2 mm and most preferably of less than 1 mm. A particular preferred range of the micrometastasis and/or the small proliferative lesions, in particular the micrometastasis, the precancerosis, the dysplasia, the metaplasia, endometriosis and/or the primary tumor(s), detectable according to the use of the present invention are between about 10 mm to about 0.2 mm, more preferably between about 8 mm to about 0.2 mm, more preferably between about 6 mm to about 0.2 mm, more preferably between about 5 mm to about 0.2 mm, more preferably between about 4 mm to about 0.2 mm, more preferably between about 3 mm to about 0.2 mm and most preferably between about 2 mm to about 0.2 mm.

Preferably the micrometastasis, which is detected according to the use of the present invention is an iatrogenic micrometastasis, a hematogenous micrometastasis, a cavitary micrometastasis, an intraluminal micrometastasis, a lymphatic micrometastasis, a local micrometastasis, and/or a regional micrometastasis.

The micrometastasis diagnosed preferably originates from a primary tumor including but not limited to malignomas (e.g., carcinomas, sarcomas) of the gastrointestinal or colorectal tract, liver, pancreas, kidney, bladder, prostate, endometrium, ovary, testes, melanoma, dysplastic oral mucosa, invasive oral cancers, small cell and non-small cell lung carcinomas; mammary tumors, e.g. a hormone-dependent breast cancers, hormone independent breast cancers; transitional and squamous cell cancers; neurological malignancies including neuroblastoma, gliomas, astrocytomas, osteosarcomas; soft tissue sarcomas; hemangioamas and endocrinological tumors. The small primary tumor detectable according to the use of the present invention preferably is one of the above indicated tumors. In a particular preferred embodiment the small primary tumor or the micrometastasis is a mammary tumor, in particular a hormone-dependent breast cancer or hormone independent breast cancer.

The precancerosis, which is detectable according to the use of the present invention is preferably selected from the group consisting of precancerosis of the skin, in particular actinic keratosis, cutaneaous horn, actinic cheilitis, tar keratosis, arsenic keratosis, x-ray keratosis, Bowen's disease, bowenoid papulosis, lentigo maligna, lichen sclerosus, and lichen rubber mucosae; precancerosis of the digestive tract, in particular erythroplakia, leukoplakia, Barrett's esophagus, Plummer-Vinson syndrome, crural ulcer, gastropathia hypertrophica gigantea, borderline carcinoma, neoplastic intestinal polyp, rectal polyp, porcelain gallbladder; gynaecological precancerosis, in particular carcinoma ductale in situ (CDIS), cervical intraepithelial neoplasia (CIN), leukoplakia, endometrial hyperplasia (grade III), vulvar dystrophy, vulvar intraepithelial neoplasia (VIN), hydatidiform mole; urologic precancerosis, in particular bladder papillomatosis, Queyrat's erythroplasia, testicular intraepithelial neoplasia (TIN), leukoplakia; carcinoma in situ (CIS); precancerosis caused by chronic inflammation, in particular pyoderma, osteomyelitis, acne conglobata, lupus vulgaris, and fistula.

Dysplasia is frequently a forerunner of cancer, and is found mainly in the epithelia; it is the most disorderly form of non-neoplastic cell growth, involving a loss in individual cell uniformity and in the architectural orientation of cells. Dysplastic cells often have abnormally large, deeply stained nuclei, and exhibit pleomorphism. Dysplasia characteristically occurs where there exist chronic irritation or inflammation. Dysplastic disorders which can be diagnosed according to the present invention include, but are not limited to, anhidrotic ectodermal dysplasia, anterofacial dysplasia, asphyxiating thoracic dysplasia, atriodigital dysplasia, bronchopulmonary dysplasia, cerebral dysplasia, cervical dysplasia, chondroectodermal dysplasia, cleidocranial dysplasia, congenital ectodermal dysplasia, craniodiaphysial dysplasia, craniocarpotarsal dysplasia, craniometaphysial dysplasia, dentin dysplasia, diaphysial dysplasia, ectodermal dysplasia, enamel dysplasia, encephalo-ophthalmic dysplasia, dysplasia epiphysialis heminelia, dysplasia epiphysialis multiplex, dysplasia epiphysalis punctata, epithelial dysplasia, faciodigitogenital dysplasia, familial fibrous dysplasia of jaws, familial white folded dysplasia, fibromuscular dysplasia, fibrous dysplasia of bone, florid osseous dysplasia, hereditary renal-retinal dysplasia hidrotic ectodermal dysplasia, hypohidrotic ectodermal dysplasia, lymphopenic thymic dysplasia, mammary dysplasia, mandibulofacial dysplasia, metaphysical dysplasia, Mondini dysplasia, monostotic fibrous dysplasia, mucoepithelial dysplasia, multiple epiphysial dysplasia, oculoauriculovertebral dysplasia, oculodentodigital dysplasia, oculovertebral dysplasia, odontogenic dysplasia, ophthalmomandibulomelic dysplasia, periapical cemental dysplasia, polyostotic fibrous dysplasia, pseudoachondroplastic spondyloepiphysial dysplasia, retinal dysplasia, septo-optic dysplasia, spondyloepiphysial dysplasia, and ventriculoradial dysplasia.

Metaplasia is a form of controlled cell growth in which one type of adult or fully differentiated cell substitutes for another type of adult cell. Metaplastic disorders, which are detectable according to the use of the present invention are preferably selected from the group consisting of agnogenic myeloid metaplasia, apocrine metaplasia, atypical metaplasia, autoparenchymatous metaplasia, connective tissue metaplasia, epithelial metaplasia, intestinal metaplasia, metaplastic anemia, metaplastic ossification, metaplastic polyps, myeloid metaplasia, primary myeloid metaplasia, secondary myeloid metaplasia, squamous metaplasia, squamous metaplasia of amnion, symptomatic myeloid metaplasia and regenerative metaplasia.

The ocular disease, which is detectable according to the use of the present invention is preferably selected from the group consisting of trachoma, retinopathy of prematurity, diabetic retinopathy, neovascular glaucoma and age-related macular degeneration.

Endometriosis is a gynecological disease defined by the proliferation of endometrial tissue outside the uterine cavity. Proliferating endometrial cells can distribute through the entire body and endometrial lesions have already been found in the lung and in other organs and in that respect the distribution of endometrial lesions resembles the distribution of micrometastasis. In a preferred embodiment of the use of the present invention the endometric lesions, e.g. endometrial cell clusters, which are detected are hematogenous cell clusters, cavitary cell clusters, intraluminal cell clusters, lymphatic cell clusters, local cell clusters and/or regional cell clusters.

The dose of the conjugat is not particularly limited insofar as the dose enables detection of the site to be ultimately diagnosed. It is appropriately adjusted depending on the kind of compound to be used, age, body weight and target organ or tissue and the like. Typically the dose is between 0.002 to 100 mg/kg body weight, preferably between 0.005 to 10 mg/kg body weight, more preferably between 0.01 to 2 mg/kg body weight, and most preferably between 0.02 to 1 mg/kg body weight.

The fluorescence imaging method of the present invention is practised following known methods, and each parameter, such as excitation wavelength and fluorescence wavelength to be detected, can appropriately be determined for each conjugate to be administered, to achieve optimal imaging and resolution. The time spend from administration of the conjugates to the determination by the fluorescence imaging method varies depending on the conjugate and the administration target. For example, when the conjugate is used for tumor imaging the lapse time typically will be in the range of about 2 to 120 hours after administration and preferably between about 2 to about 10 h after administration. When the lapse time is too short the fluorescence is so intense that angiogenic and non-angiogenic tissues can not be clearly differentiated (low signal-to-noise ratio). Devices for the fluorescence imaging method are well known in the art and are describe in, for example, EP 0 868 143, EP 1 146 811 A1, EP 1 408 824 A2, EP 1 409 995 A1, and EP 1 410 330 A2.

As has been outlined above the present invention can also be used in connection with surgical procedures and, therefore, the detection of the fluorescence can be carried out using surgical microscopes, microscopes, magnifying glasses and the like. Such devices can be employed both in a variety of surgical procedures including open and endoscopic procedures. It is also possible to use the invention in connection with devices and procedures, which are commonly used for routine screening for cancers, e.g. colonoscopy and gastroscopy.

### Brief Description of the Figure

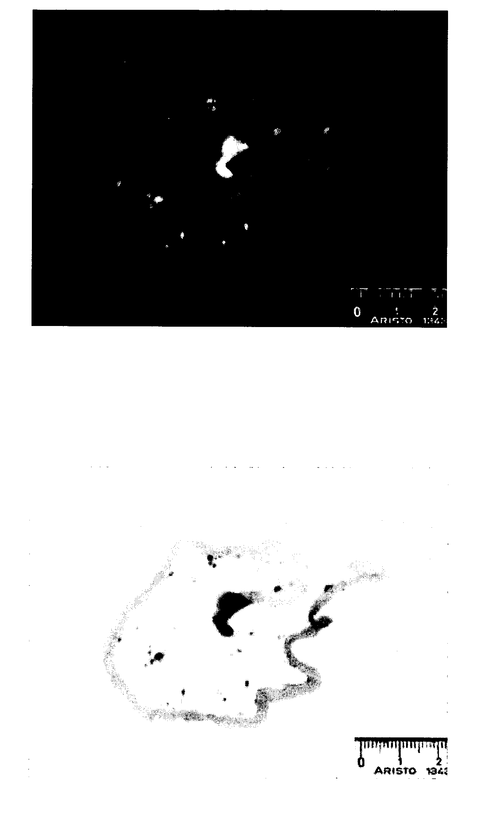

**Fig. 1:** The effectiveness of the dye conjugate in mesenterial Capan-1 micrometastasis 6 h after substance administration. The upper panel depicts the original image, while the lower panel depicts the inverted image. White and black dots or areas, respectively, show micrometastasis.

### Examples

### Examples 1-16: Synthesis of Indotricarbocyanine Dyes with Maleimide Groups

### Example 1: Trisodium 3,3-dimethyl-2-{7-[3,3-dimethyl-5-sulfonato-1-(2-sulfonatoethyl)-3H-indolium-2-yl]-4-(2-{[2-(2,5-dioxo-2,5-dihydro-1H-pyrrol-1-yl)ethyl]carbamoyl}ethyl)hepta-2,4,6-trien-1-ylidene}-1-(2-sulfonatoethyl)-2,3-dihydro-1H-indole-5-sulfonate, internal salt (Formula XVIII)

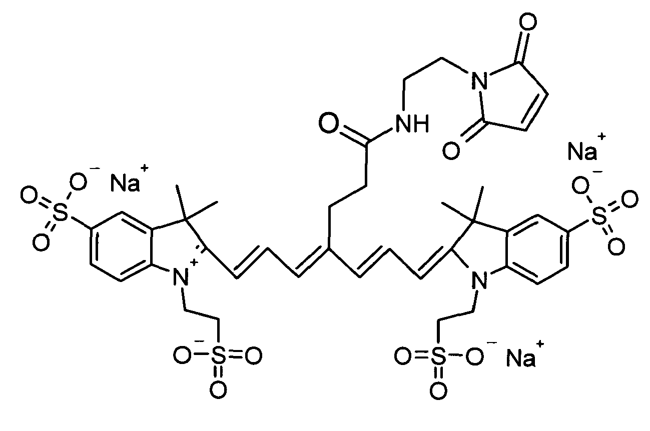

### a) 1-(2-Sulfonatoethyl)-2,3,3-trimethyl-3H-indolenine-5-sulfonic acid, internal salt

10 g (0.04 mol) of 2,3,3-trimethyl-3H-indolenine-5-sulfonic acid (Bioconjugate Chem 1993, 4, 105)*,* 6.8 g (0.04 mol) of 2-chloroethanesulfonic acid chloride and 4.2 g (0.04 mol) of triethylamine are refluxed in 200 ml of acetonitrile for 6 hours. The precipitate is suctioned off and dried. Yield 5.0 g (35% of theory). Anal Biochem 1994, 217, 197

### b) 3-Pyridin-4-yl-propionic acid-tert-butyl ester

20 g (89 mmol) of t-butyl-P,P-dimethylphosphonoacetate in 50 ml of THF is added in drops at 0°C to a suspension of 3.9 g (98 mmol) of sodium hydride (60 % in mineral oil) in 250 ml of THF. After 1 hour of stirring at 0°C, a solution of 10 g (93 mmol) of pyridine-4-carbaldehyde in 50 ml of tetrahydrofuran is added in drops, and the reaction mixture is stirred for 1 hour at 0°C and for 18 hours at room temperature. The precipitated solid is removed by filtration, and the solution is concentrated by evaporation. The residue is dissolved in isopropanol while being heated, non-soluble portions are filtered off, and the solution is cooled to 0°C for crystallization. The solid that is produced is filtered off, stirred with hexane, filtered and dried. The intermediate product (15.3 g) is hydrogenated in 150 ml of ethanol with 0.15 g of 10% palladium/activated carbon for 6 hours. The catalyst is filtered off, the solution is concentrated by evaporation, and the residue is filtered on silica gel (mobile solvent diethyl ether). 13.0 g of a light yellow oil (71 % of theory) is obtained.

### c) 3-[2-(tert-Butyloxycarbonyl)ethyl]glutaconaldehyde-dianilide-hydrobromide

A solution of 10 g (48 mmol) of 3-pyridin-4-yl-propionic acid-*tert*-butyl ester in 150 ml of diethyl ether is mixed with 8.9 g (96 mmol) of aniline and then mixed at 0°C with a solution of 5.4 g (48 mmol) of bromocyanogen in 2 ml of diethyl ether. After 3 hours of stirring at 0°C, the red solid that is produced is filtered off, washed with ether and vacuum-dried. Yield: 20.3 g (92% of theory)

### d) Trisodium 3,3-dimethyl-2-{7-[3,3-dimethyl-5-sulfonato-1-(2-sulfonatoethyl)-3H-indolium-2-yl]-4-(2-carboxyethyl) hepta-2,4,6-trien-1-ylidene}-1-(2-sulfonatoethyl)-2,3-dihydro-1H-indole-5-sulfonate, internal salt

A suspension of 1.0 g (2.2 mmol) of 3-[2-(*tert*-butyloxycarbonyl)ethyl]-glutaconaldehyde-dianilide-hydrobromide (Example 1c)) and 1.5 g (4.4 mmol) of 1-(2-sulfonatoethyl)-2,3,3-trimethyl-3H-indolenine-5-sulfonic acid (Example 1a)) in 20 ml of acetic acid anhydride and 5 ml of acetic acid is mixed with 0.75 g (9.1 mmol) of sodium acetate and stirred for 1 hour at 120°C. After cooling, it is mixed with diethyl ether, the precipitated solid is filtered off and purified by chromatography (RP-C18-silica gel, mobile solvent water/methanol) and the product is freeze-dried (0.5 g). The cleavage of the protective group is carried out by stirring the intermediate product in 4 ml of dichloromethane/1 ml of trifluoroacetic acid for 1 hour. After concentration by evaporation and chromatographic purification (RP-C18-silica gel, mobile solvent water/methanol), 0.45 g (23% of theory) of a blue lyophilizate is obtained.

### e) Trisodium 3,3-dimethyl-2-{7-[3,3-dimethyl-5-sulfonato-1-(2-sulfonatoethyl)-3H-indolium-2-yl]-4-(2-{ [2-(2,5-dioxo-2,5-dihydro-1H-pyrrol-1-yl)ethyl]carbamoyl}ethyl)hepta-2,4,6-trien-1-ylidene}-1-(2-sulfonatoethyl)-2,3-dihydro-1H-indole-5-sulfonate, internal salt

0.4 g (0.45 mmol) of the title compound of Example 1d) and 45 mg (0.45 mmol) of triethylamine are dissolved in 10 ml of dimethylformamide, mixed at 0°C with 0.15 g (0.45 mmol) of TBTU and stirred for 10 minutes. Then, a solution of 0.17 g (0.68 mmol) of N-(2-aminoethyl)maleimide-trifluoroacetate (Int J Pept Protein Res 1992, 40, 445) and 68 mg (0.68 mmol) of triethylamine in 0.5 ml of dimethylformamide is added, and it is stirred for 1 hour at room temperature. After 10 ml of diethyl ether is added, the solid is centrifuged off, dried and purified by means of chromatography (RP C-18 silica gel, gradient methanol/water).

Yield: 0.30 g of a blue lyophilizate (65% of theory).

| | | | | | | |
|---|---|---|---|---|---|---|
| Elementary analysis: | Cld.: | C 47.24 | H 4.26 | N 5.51 | S 12.61 | Na 6.78 |
| | Fnd.: | C 47.74 | H 4.47 | N 5.40 | S 11.99 | Na 7.02 |

### Example 2: Trisodium 3,3-dimethyl-2-{7-[3,3-dimethyl-5-sulfonato-1-(2-sulfonatoethyl)-3H-indolium-2-yl]-4-(2-{[6-(2,5-dioxo-2,5-dihydro-1H-pyrrol-1-yl)hexyl]carbamoyl}ethyl)hepta-2,4,6-trien-1-ylidene}-1-(2-sulfonatoethyl)-2,3-dihydro-1H-indole-5-sulfonate, internal salt (Formula XIX)

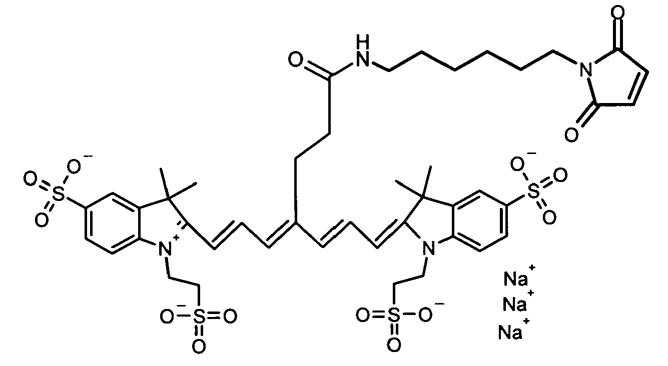

The synthesis is carried out analogously to Example 1e) from 0.4 g (0.45 mmol) of the title compound of Example 1d) and 0.21 g (0.68 mmol) of N-(6-aminohexyl)maleimide-trifluoroacetate (Int J Pept Protein Res 1992, 40, 445). Yield: 0.38 g of a blue lyophilizate (81% of theory).

| | | | | | | |
|---|---|---|---|---|---|---|
| Elementary analysis: | Cld.: | C 49.25 | H 4.79 | N 5.22 | S 11.95 | Na 6.43 |
| | Fnd.: | C 48.96 | H 4.92 | N 5.32 | S 11.88 | Na 6.56 |

### Example 3: Trisodium 3,3-dimethyl-2-{7-[3,3-dimethyl-5-sulfonato-1-(2-sulfonatoethyl)-3H-indolium-2-yl]-4-(2-{[13-(2,5-dioxo-2,5-dihydro-1H-pyrrol-1-yl)-4,7,10-trioxatridecyl]carbamoyl}ethyl)hepta-2,4,6-trien-1-ylidene}-1-(2-sulfonatoethyl)-2,3-dihydro-1H-indole-5-sulfonate, internal salt (Formula XX)

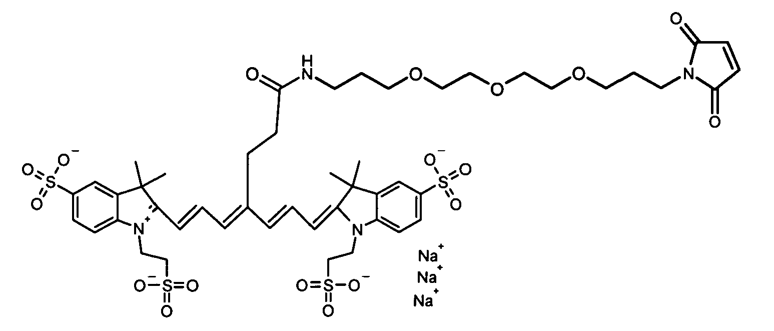

The synthesis is carried out analogously to Example 1e) from 0.4 g (0.45 mmol) of the title compound of Example 1d) and 0.28 g (0.68 mmol) of N-(13-amino-4,7,10-trioxatridecyl)maleimide-trifluoroacetate (Int JPept Protein Res 1992, 40, 445). Yield: 0.27 g of a blue lyophilizate (51% of theory).

| | | | | | | |
|---|---|---|---|---|---|---|
| Elementary analysis: | Cld.: | C 48.97 | H 5.05 | N 4.76 | S 10.89 | Na 5.86 |
| | Fnd.: | C 49.22 | H 5.16 | N 4.62 | S 10.67 | Na 5.66 |

### Example 4: Trisodium 3,3-dimethyl-2-{7-[3,3-dimethyl-5-sulfonato-1-(2-sulfonatoethyl)-3H-indolium-2-yl]-4-(4-{[2-(2,5-dioxo-2,5-dihydro-1H-pyrrol-1-yl)ethyl]carbamoyl}-butyl)hepta-2,4,6-trien-1-ylidene}-1-(2-sulfonatoethyl)-2,3-dihydro-1H-indole-5-sulfonate, internal salt (Formula XXI)

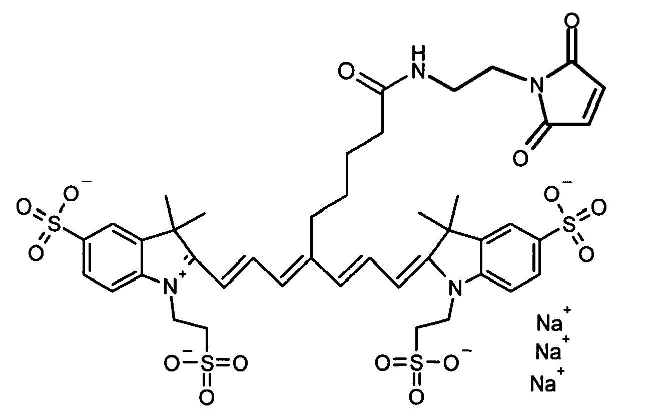

### a) (3-tert-Butoxycarbonyl-propyl)-triphenyl-phosphonium bromide

50 g (0.30 mol) of 4-bromobutyric acid is mixed drop by drop in 400 ml of THF at - 40°C with 187 g (0.89 mol) of trifluoroacetic acid anhydride. After 30 minutes of stirring at - 40°C, 400 ml of tert-butanol/30 ml of THF is added in drops within 1 hour. After 16 hours of stirring at room temperature, the reaction mixture is poured onto an ice-cooled sodium carbonate solution, the aqueous phase is extracted three times with diethyl ether, and the organic phases are dried on sodium sulfate and concentrated by evaporation. The residue is distilled in a vacuum (boiling point 72°C/0.9 mbar; yield: 41 g). The reaction to form phosphonium salt is carried out by reflux-heating 41 g (0.18 mol) of intermediate product, 44.6 g (0.17 mol) of triphenylphosphine and 32.5 g (0.36 mol) of sodium bicarbonate in 250 ml of acetonitrile for 20 hours. The reaction mixture is filtered, concentrated by evaporation, and the residue is brought to crystallization by stirring with diethyl ether. Yield: 58.5 g (40% of theory, relative to 4-bromobutyric acid) of a white solid.

### b) 5-Pyridin-4-yl-pentanoic acid-t-butyl ester

A solution of 14 g (28 mmol) of (3-*tert*-butoxycarbonyl-propyl)-triphenyl-phosphonium bromide (Example 4a)) in 100 ml of anhydrous THF is mixed at -40°C in an air-free environment within 20 minutes with 17.5 ml (28 mmol) of butyllithium (1.6 M in hexane) and stirred for 1 hour at -40°C. A solution of 2.78 g (26 mmol) of 4-pyridinecarbaldehyde in 20 ml of THF is added in drops and stirred for 16 hours at room temperature, then poured onto ice water, the aqueous phase is extracted three times with diethyl ether, and the organic phases are dried on sodium sulfate and concentrated by evaporation. After chromatographic purification (silica gel, mobile solvent hexane/ethyl acetate), the product is obtained as an E,Z-mixture (4:1 after ¹H-NMR; 5.0 g). To hydrogenate the double bond, the intermediate product is dissolved in 200 ml of methanol and stirred with 100 mg of PtO₂ catalyst at room temperature over hydrogen. After filtration and concentration by evaporation, a yellow oil is obtained. Yield: 4.9 g (74% of theory).

### c) 3-[4-(tert-Butyloxycarbonyl)butyl]glutaconaldehyde-dianilide-hydrobromide

A solution of 4.0 g (17 mmol) of 5-pyridin-4-yl-pentanoic acid-t-butylester in 35 ml of diethyl ether is mixed with 3.2 g (34 mmol) of aniline and then at 0°C with a solution of 1.9 g (17 mmol) of bromocyanogen in 8 ml of diethyl ether. After 3 hours of stirring at 0°C, the red solid that is produced is filtered off, washed with ether and vacuum-dried. Yield: 7.8 g (95% of theory).

### d) Trisodium 3,3-dimethyl-2-{7-[3,3-dimethyl-5-sulfonato-1-(2-sulfonatoethyl)-3H-indolium-2-yl]-4-(4-carboxybutyl)hepta-2,4,6-trien-1-ylidene}-1-(2-sulfonatoethyl)-2,3-dihydro-1H-indole-5-sulfonate, internal salt

The synthesis is carried out analogously to Example 1d) from the title compound of Example 4c) (2.5 mmol) and 1-(2-sulfonatoethyl)-2,3,3-trimethyl-3H-indolenine-5-sulfonic acid (5 mmol). Yield: 0.85 g (37% of theory) of a blue lyophilizate.

### e) Trisodium 3,3-dimethyl-2-{7-[3,3-dimethyl-5-sulfonato-1-(2-sulfonatoethyl)-3H-indolium-2-yl]-4-(4-{[2-(2,5-dioxo-2,5-dihydro-1H-pyrrol-1-yl)ethyl]carbamoyl}-butyl)hepta-2,4,6-trien-1-ylidene}-1-(2-sulfonatoethyl)-2,3-dihydro-1H-indole-5-sulfonate, internal salt

The synthesis is carried out analogously to Example 1e) from 0.4 g (0.43 mmol) of the title compound of Example 4d). Yield: 0.31 g (69% of theory) of a blue lyophilizate.

| | | | | | | |
|---|---|---|---|---|---|---|
| Elementary analysis: | Cld.: | C 48.27 | H 4.53 | N 5.36 | S 12.27 | Na 6.60 |
| | Fnd.: | C 48.01 | H 4.44 | N 5.56 | S 12.10 | Na 6.81 |

### Example 5: Trisodium 3,3-dimethyl-2-{7-[3,3-dimethyl-5-sulfonato-1-(2-sulfonatoethyl)-3H-indolium-2-yl]-4-(4-{[6-(2,5-dioxo-2,5-dihydro-1H-pyrrol-1-yl)hexyl]carbamoyl}butyl)hepta-2,4,6-trien-1-ylidene}-1-(2-sulfonatoethyl)-2,3-dihydro-1H-indole-5-sulfonate, internal salt (Formula XXII)

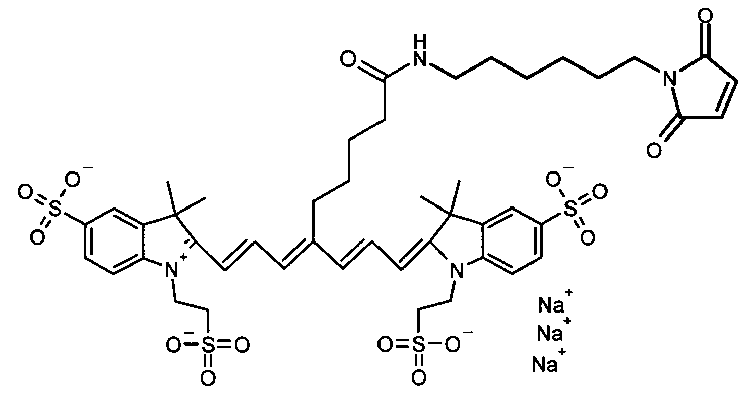

The synthesis is carried out analogously to Example 1e) from 0.4 g (0.43 mmol) of the title compound of Example 4d) and 0.20 g (0.66 mmol) of N-(6-aminohexyl)maleimide-trifluoroacetate. Yield: 0.35 g of a blue lyophilizate (74% of theory).

| | | | | | | |
|---|---|---|---|---|---|---|
| Elementary analysis: | Cld.: | C 50.17 | H 5.03 | N 5.09 | S 11.65 | Na 6.26 |
| | Fnd.: | C 49.83 | H 4.89 | N 5.34 | S 12.05 | Na 6.42 |

### Example 6: Trisodium 3,3-dimethyl-2-{7-[3,3-dimethyl-5-sulfonato-1-(2-sulfonatoethyl)-3H-indolium-2-yl]-4-(4-{[13-(2,5-dioxo-2,5-dihydro-1H-pyrrol-1-yl)-4,7,10-trioxatridecyl]carbamoyl}butyl)hepta-2,4,6-trien-1-ylidene}-1-(2-sulfonatoethyl)-2,3-dihydro-1H-indole-5-sulfonate, internal salt (Formula XXIII)

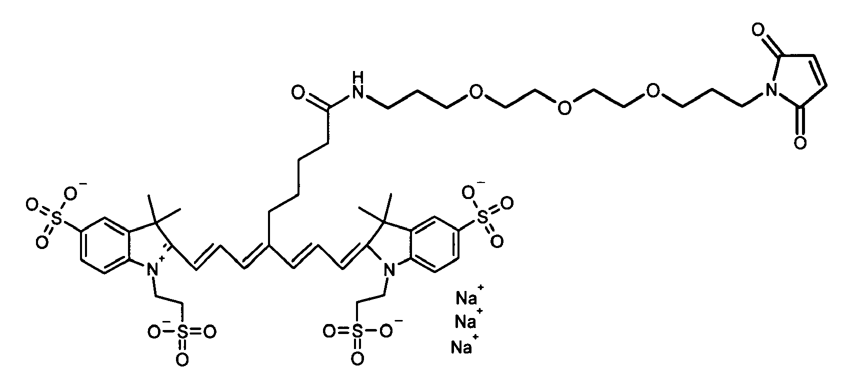

The synthesis is carried out analogously to Example 1e) from 0.4 g (0.43 mmol) of the title compound of Example 1d) and 0.30 g (0.72 mmol) of N-(13-amino-4,7,10-trioxatridecyl)maleimide-trifluoracetate. Yield: 0.27 g of a blue lyophilizate (52% of theory).

| | | | | | | |
|---|---|---|---|---|---|---|
| Elementary analysis: | Cld.: | C 49.83 | H 5.27 | N 4.65 | S 10.64 | Na 5.72 |
| | Fnd.: | C 49.45 | H 5.19 | N 4.66 | S 10.85 | Na 5.80 |

### Example 7: Trisodium 3,3-dimethyl-2-{7-[3,3-dimethyl-5-sulfonato-1-(2-sulfonatoethyl)-3H-indolium-2-yl]-4-(6-{[2-(2,5-dioxo-2,5-dihydro-1H-pyrrol-1-yl)ethyl]carbamoyl}hexyl)hepta-2,4,6-trien-1-ylidene}-1-(2-sulfonatoethyl)-2,3-dihydro-1H-indole-5-sulfonate, internal salt (Formula XXIV)

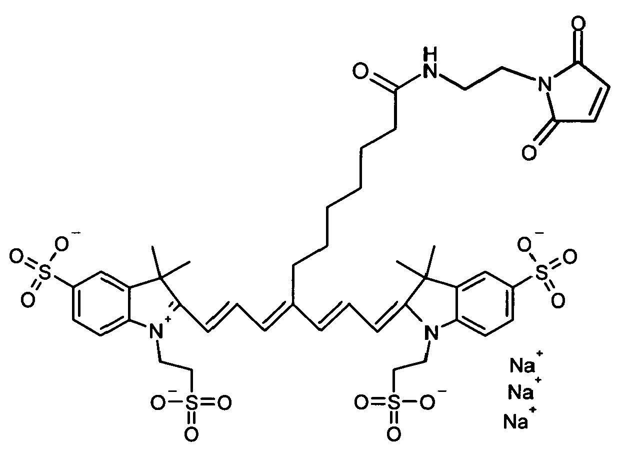

### a) (3-tert-Butoxycarbonyl-pentyl)-triphenyl-phosphonium bromide

The production is carried out as described in Example 4a), whereby the intermediate product 6-bromohexanoic acid-*tert*-butyl ester is reacted as a crude product. 79 g of product (69% of theory) is obtained as a viscous, colorless oil from 50 g of 6-bromohexanoic acid.

### b) 7-Pyridin-4-yl-heptanoic acid-t-butyl ester

The production is carried out as described in Example 4b). 7.5 g of 7-pyridin-4-yl-heptanoic acid-t-butyl ester (65% of theory) is obtained as a yellow oil from 25 g (48.7 mmol) of (3-*tert*-butoxycarbonyl-pentyl)-triphenyl-phosphonium bromide (Example 7a).

### c) 3-[6-(tert-Butyloxycarbonyl)hexyl]glutaconaldehyde-dianilide-hydrobromide

A solution of 5.0 g (19 mmol) of 7-pyridin-4-yl-heptanoic acid-t-butyl ester in 30 ml of diethyl ether is mixed with 3.6 g (38 mmol) of aniline and then at 0°C with a solution of 2.1 g (19 mmol) of bromocyanogen in 5 ml of diethyl ether. After 2.5 hours of stirring at 0°C, the red solid that is produced is filtered off, washed with ether and vacuum-dried. Yield: 8.9 g (91% of theory).

### d) Trisodium 3,3-dimethyl-2-{7-[3,3-dimethyl-5-sulfonato-1-(2-sulfonatoethyl)-3H-indolium-2-yl]-4-(6-carboxyhexyl)hepta-2,4,6-trien-1-ylidene}-1-(2-sulfonatoethyl)-2,3-dihydro-1H-indole-5-sulfonate, internal salt

The synthesis is carried out analogously to Example 1d) from the title compound of Example 7c) (3 mmol) and 1-(2-sulfonatoethyl)-2,3,3-trimethyl-3H-indolenine-5-sulfonic acid (6 mmol). Yield: 1.5 g (54% of theory) of a blue lyophilizate.

### e) Trisodium 3,3-dimethyl-2-{7-[3,3-dimethyl-5-sulfonato-1-(2-sulfonatoethyl)-3H-indolium-2-yl]-4-(6-{[2-(2,5-dioxo-2,5-dihydro-1H-pyrrol-1-yl)ethyl]carbamoyl}hexyl)hepta-2,4,6-trien-1-ylidene}-1-(2-sulfonatoethyl)-2,3-dihydro-1H-indole-5-sulfonate, internal salt

The synthesis is carried out analogously to Example 1e) from 0.4 g (0.43 mmol) of the title compound of Example 7d). Yield: 0.31 g (69% of theory) of a blue lyophilizate.

| | | | | | | |
|---|---|---|---|---|---|---|
| Elementary analysis: | Cld.: | C 49.25 | H 4.79 | N 5.22 | S 11.95 | Na 6.43 |
| | Fnd.: | C 48.98 | H 4.86 | N 5.12 | S 11.76 | Na 6.77 |

### Example 8: Trisodium 3,3-dimethyl-2-{7-[3,3-dimethyl-5-sulfonato-1-(2-sulfonatoethyl)-3H-indolium-2-yl]-4-(6-{[6-(2,5-dioxo-2,5-dihydro-1H-pyrrol-1-yl)hexyl]carbamoyl}hexyl)hepta-2,4,6-trien-1-ylidene}-1-(2-sulfonatoethyl)-2,3-dihydro-1H-indole-5-sulfonate, internal salt (Formula XXV)

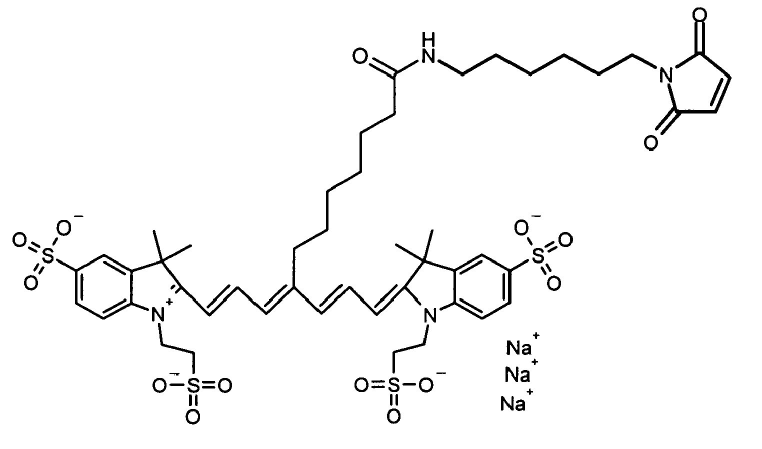

The synthesis is carried out analogously to Example 1e) from 0.5 g (0.53 mmol) of the title compound of Example 7d) and 0.23 g (0.75 mmol) of N-(6-aminohexyl)maleimide-trifluoroacetate. Yield: 0.42 g of a blue lyophilizate (70% of theory).

| | | | | | | |
|---|---|---|---|---|---|---|
| Elementary analysis: | Cld.: | C 51.05 | H 5.27 | N 4.96 | S 11.36 | Na 6.11 |
| | Fnd.: | C 50.74 | H 5.55 | N 4.76 | S 11.38 | Na 6.35 |

### Example 9: Trisodium 3,3-dimethyl-2-{7-[3,3-dimethyl-5-sulfonato-1-(2-sulfonatoethyl)-3H-indolium-2-yl]-4-(6-{[13-(2,5-dioxo-2,5-dihydro-1H-pyrrol-1-yl)-4,7,10-trioxatridecyl]carbamoyl}hexyl)hepta-2,4,6-trien-1-ylidene}-1-(2-sulfonatoethyl)-2,3-dihydro-1H-indole-5-sulfonate, internal salt (Formula XXVI)

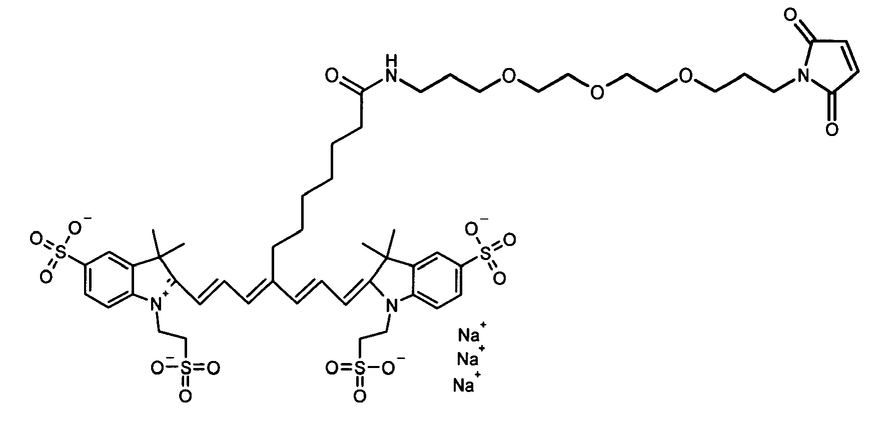

The synthesis is carried out analogously to Example 1e) from 0.5 g (0.53 mmol) of the title compound of Example 7d) and 0.44 g (1.06 mmol) of N-(13-amino-4,7,10-trioxatridecyl)maleimide-trifluoroacetate. Yield: 0.24 g of a blue lyophilizate (37% of theory).

| | | | | | | |
|---|---|---|---|---|---|---|
| Elementary analysis: | Cld.: | C 50.64 | H 5.48 | N 4.54 | S 10.40 | Na 5.59 |
| | Fnd.: | C 50.30 | H 5.56 | N 4.34 | S 10.15 | Na 5.73 |

### Example 10: Trisodium 3,3-dimethyl-2-{7-[3,3-dimethyl-5-sulfonato-1-(2-sulfonatoethyl)-3H-indolium-2-yl]-4-(5-{[2-(2,5-dioxo-2,5-dihydro-1H-pyrrol-1-yl)ethyl] carbamoyl}-3-oxa-pentyl)hepta-2,4,6-trien-1-ylidene}-1-(2-sulfonatoethyl)-2,3-dihydro-1H-indole-5-sulfonate, internal salt (Formula XXVII)

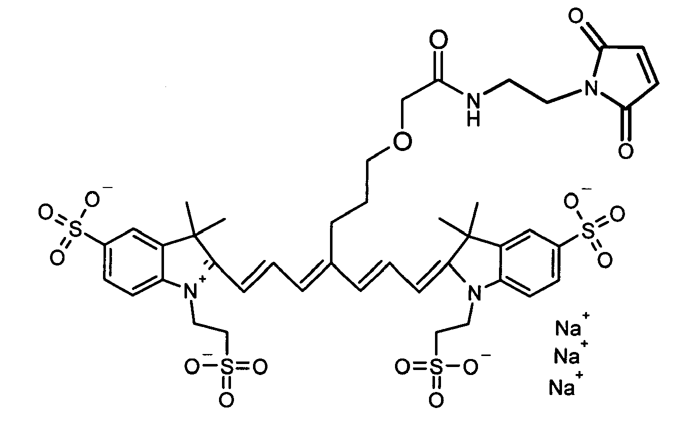

### a) 3-Oxa-6-(4-Pyridinyl)hexanoic acid-tert-butyl ester

A solution of 75 g (0.4 mol) of 3-(4-pyridinyl)-1-propanol in 400 ml of toluene/50 ml of THF is mixed with 10 g of tetrabutylammonium sulfate and 350 ml of 32% sodium hydroxide solution. Then, 123 g (0.68 mol) of bromoacetic acid-*tert*-butyl ester is added in drops and stirred for 18 hours at room temperature. The organic phase is separated, and the aqueous phase is extracted three times with diethyl ether. The combined organic phases are washed with NaCl solution, dried on sodium sulfate and concentrated by evaporation. After chromatographic purification (silica gel: mobile solvent hexane:ethyl acetate), 56 g of product (41% of theory) is obtained as a brownish oil.

### b) 3-[4-Oxa-5-(tert-butyloxycarbonyl)pentyl]glutaconaldehyde-dianilide-hydrobromide

A solution of 5.0 g (20 mmol) of 3-oxa-6-(4-pyridinyl)hexanoic acid-*tert*-butyl ester in 60 ml of diethyl ether is mixed with 3.7 g (40 mmol) of aniline and then at 0°C with a solution of 2.2 g (20 mmol) of bromocyanogen in 8 ml of diethyl ether. After 1 hour of stirring at 0°C, 50 ml of diethyl ether is mixed, and the red solid that is produced is filtered off, washed with ether and vacuum-dried. Yield: 8.5 g (85% of theory) of a violet solid.

### c) Trisodium 3,3-dimethyl-2-{7-[3,3-dimethyl-5-sulfonato-1-(2-sulfonatoethyl)-3H-indolium-2-yl]-4-(6-carboxy-4-oxahexyl)hepta-2,4,6-trien-1-ylidene }-1-(2-sulfonatoethyl)-2,3-dihydro-1H-indole-5-sulfonate, internal salt

A suspension of 3.0 g (6 mmol) of 3-[2-(*tert*-butyloxycarbonyl)ethyl]-glutaconaldehyde-dianilide-hydrobromide (Example 10b)) and 4.2 g (12 mmol) of 1-(2-sulfonatoethyl)-2,3,3-trimethyl-3H-indolenine-5-sulfonic acid (Example 1a)) in 50 ml of acetic acid anhydride and 10 ml of acetic acid is mixed with 2.5 g (30 mmol) of sodium acetate and stirred for 50 minutes at 120°C. After cooling, it is mixed with diethyl ether, the precipitated solid is filtered off, absorptively precipitated in acetone and dried under high vacuum. After chromatographic purification (RP-C18-silica gel, mobile solvent water/methanol), removal of the methanol in a vacuum and freeze-drying, the title compound is immediately obtained. Yield: 2.3 g (41% of theory) of a blue lyophilizate.

### d) Trisodium 3,3-dimethyl-2-{7-[3,3-dimethyl-5-sulfonato-1-(2-sulfonatoethyl)-3H-indolium-2-yl]-4-(5-{[2-(2,5-dioxo-2,5-dihydro-1H-pyrrol-1-yl)ethyl]carbamoyl}-3-oxa-pentyl)hepta-2,4,6-trien-1-ylidene}-1-(2-sulfonatoethyl)-2,3-dihydro-1H-indole-5-sulfonate, internal salt

The synthesis is carried out analogously to Example 1c) from 1.0 g (1.1 mmol) of the title compound of Example 10c). Yield: 0.85 g (73% of theory) of a blue lyophilizate.

| | | | | | | |
|---|---|---|---|---|---|---|
| Elementary analysis: | Cld.: | C 47.54 | H 4.46 | N 5.28 | S 12.09 | Na 6.50 |
| | Fnd.: | C 47.97 | H 4.65 | N 5.10 | S 12.02 | Na 6.68 |

### Example 11: Trisodium 3,3-dimethyl-2-{7-[3,3-dimethyl-5-sulfonato-1-(2-sulfonatoethyl)-3H-indolium-2-yl]-4-(5-{[6-(2,5-dioxo-2,5-dihydro-1H-pyrrol-1-yl)hexyl]carbamoyl}-3-oxa-pentyl)hepta-2,4,6-trien-1-ylidene}-1-(2-sulfonatoethyl)-2,3-dihydro-1H-indole-5-sulfonate, internal salt (Formula XXVIII)

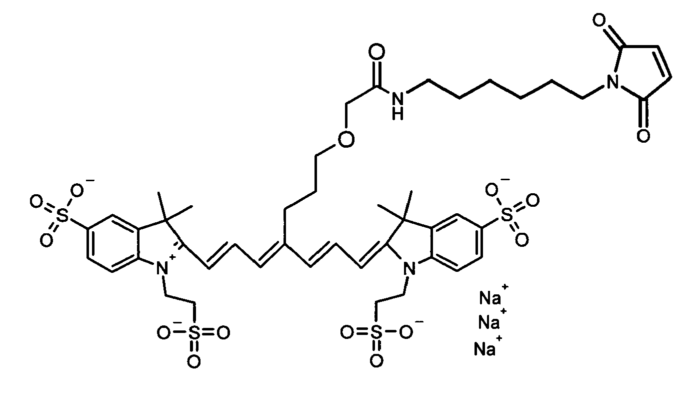

The synthesis is carried out analogously to Example 1e) from 0.5 g (0.55 mmol) of the title compound of Example 10c) and 0.23 g (0.75 mmol) of N-(6-aminohexyl)maleimide-trifluoroacetate. Yield: 0.42 g of a blue lyophilizate (68% of theory).

| | | | | | | |
|---|---|---|---|---|---|---|
| Elementary analysis: | Cld.: | C 49.46 | H 4.96 | N 5.01 | S 11.48 | Na 6.17 |
| | Fnd.: | C 48.95 | H 5.21 | N 5.22 | S 11.23 | Na 6.60 |

### Example 12: Trisodium 3,3-dimethyl-2-{7-[3,3-dimethyl-5-sulfonato-1-(2-sulfonatoethyl)-3H-indolium-2-yl]-4-(5-{[13-(2,5-dioxo-2,5-dihydro-1H-pyrrol-1-yl)-4,7,10-trioxatridecyl]carbamoyl}-4-oxapentyl)hepta-2,4,6-trien-1-ylidene}-1-(2-sulfonatoethyl)-2,3-dihydro-1H-indole-5-sulfonate, internal salt (Formula XXIX)

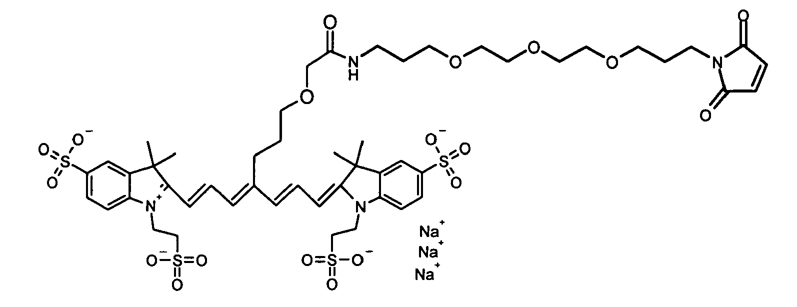

The synthesis is carried out analogously to Example 1e) from 0.5 g (0.55 mmol) of the title compound of Example 10c) and 0.46 g (1.06 mmol) of N-(13-amino-4,7,10-trioxatridecyl)maleimide-trifluoroacetate. Yield: 0.34 g of a blue lyophilizate (56% of theory).

| | | | | | | |
|---|---|---|---|---|---|---|
| Elementary analysis: | Cld.: | C 49.17 | H 5.20 | N 4.59 | S 10.50 | Na 5.65 |
| | Fnd.: | C 49.34 | H 5.32 | N 4.45 | S 10.28 | Na 5.56 |

### Example 13: Trisodium 3,3-dimethyl-2-[2-(1-{[3,3-dimethyl-5-sulfonato-1-(2-sulfonatoethyl)-3H-indolium-2-yl]vinylene}-2-[4-(2-{[2-(2,5-dioxo-2,5-dihydro-1H-pyrrol-1-yl)ethyl]carbamoyl}ethyl)-phenoxy]cyclohex-1-en-3-yliden)ethylidene]-1-(2-sulfonatoethyl)-2,3-dihydro-1H-indole-5-sulfonate, internal salt (Formula XXX)

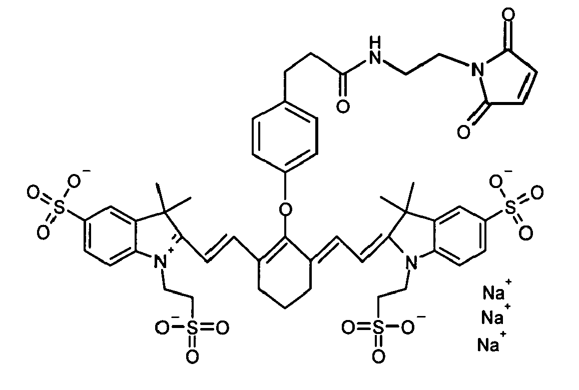

### a) Trisodium 3,3-dimethyl-2-[2-(1- {[3,3-dimethyl-5-sulfonato-1-(2-sulfonatoethyl)-3H-indolium-2-yl]vinylene }-2-chloro-cyclohex-1-en-3-ylidene)ethylidene]-1-(2-sulfonatoethyl)-2,3-dihydro-1H-indole-5-sulfonate, internal salt

5.0 g (14.4 mmol) of 1-(2-sulfonatoethyl)-2,3,3-trimethyl-3H-indolenine-5-sulfonic acid (Example 1a) and 2.6 g (7.2 mmol) of N-[(3-(anilinomethylene)-2-chloro-1-cyclohexen-1-yl)methylene]aniline hydrochloride (*Aldrich Company*) are refluxed together with 2.5 g (30 mmol) of anhydrous sodium acetate in 100 ml of methanol for 1 hour, cooled, mixed with 150 ml of diethyl ether and stirred overnight. The precipitate is suctioned off, dried and purified by chromatography (silica gel, gradient: dichloromethane/methanol). Yield: 3.8 g (58% of theory) of a blue solid.

### b) Trisodium 3,3-dimethyl-2-[2-(1-{[3,3-dimethyl-5-sulfonato-1-(2-sulfonatoethyl)-3H-indolium-2-yl]vinylene}-2-[4-(2-carboxyethyl)phenoxy]cyclohex-1-en-3-ylidene)ethylidene]-1-(2-sulfonatoethyl)-2,3-dihydro-1H-indole-5-sulfonate, internal salt

0.37 g (2.2 mmol) of 3-(4-hydroxyphenyl)propionic acid in 30 ml of dimethylformamide is mixed with 0.18 g (4.5 mmol) of sodium hydride (60% mineral oil dispersion). After 30 minutes of stirring at room temperature, it is cooled to 0°C, a solution of 2.0 g (2.2 mmol) of the title compound of Example 12a) in 100 ml of dimethylformamide is added in drops and stirred for 2 hours at room temperature. The mixture is quenched with dry ice, and the solvent is removed in a vacuum. The residue is dissolved in methanol, stirred with 200 ml of ether, and the precipitated solid is filtered off. A chromatographic purification is carried out (silica gel, gradient: ethyl acetate/methanol). Yield: 1.9 g of a blue solid (83% of theory).

### c) Trisodium 3,3-dimethyl-2-[2-(1-{[3,3-dimethyl-5-sulfonato-1-(2-sulfonatoethyl)-3H-indolium-2-yl]vinylene}-2-[4-(2-{[2-(2,5-dioxo-2,5-dihydro-1H-pyrrol-1-yl)ethyl]carbamoyl}ethyl)-phenoxy]cyclohex-1-en-3-ylidene)ethylidene]-1-(2-sulfonatoethyl)-2,3-dihydro-1H-indole-5-sulfonate, internal salt

0.1 mg (0.10 mmol) of the title compound of Example 12b is reacted as described in Example 1e) with TBTU and N-(2-aminoethyl)maleimide-trifluoroacetate in the presence of triethylamine, and the product that is obtained is purified by chromatography. Yield: 93 mg of a blue lyophilizate (81% of theory).

| | | | | | | |
|---|---|---|---|---|---|---|
| Elementary analysis: | Cld.: | C 51.21 | H 4.47 | N 4.88 | S 11.16 | Na 6.00 |
| | Fnd.: | C 51.50 | H 4.55 | N 4.95 | S 10.93 | Na 6.15 |

### Example 14: Trisodium 3,3-dimethyl-2-[2-(1-{[3,3-dimethyl-5-sulfonato-1-(2-sulfonatoethyl)-3H-indolium-2-yl]vinylene}-2-[4-(2-{[6-(2,5-dioxo-2,5-dihydro-1H-pyrrol-1-yl)hexyl]carbamoyl}ethyl)-phenoxy]cyclohex-1-en-3-ylidene)ethylidene]-1-(2-sulfonatoethyl)-2,3-dihydro-1H-indole-5-sulfonate, internal salt (Formula XXXI)

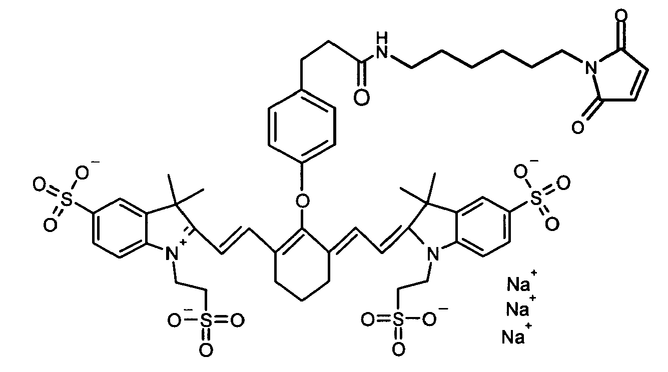

The synthesis is carried out analogously to Example 1e) from 0.7 g (0.68 mmol) of the title compound of Example 14a) and 0.53 g (1.22 mmol) of N-(13-amino-4,7,10-trioxatridecyl)maleimide-trifluoracetate. Yield: 0.56 g of a blue lyophilizate (68% of theory).

| | | | | | | |
|---|---|---|---|---|---|---|
| Elementary analysis: | Cld.: | C 48.27 | H 4.53 | N 5.36 | S 12.27 | Na 6.60 |
| | Fnd.: | C 48.01 | H 4.44 | N 5.56 | S 12.10 | Na 6.81 |

### Example 15: Trisodium 3,3-dimethyl-2-[2-(1-{[3,3-dimethyl-5-sulfonato-1-(2-sulfonatoethyl)-3H-indolium-2-yl]vinylene}-2-[4-(2-{[13-(2,5-dioxo-2,5-dihydro-1H-pyrrol-1-yl)-4,7,10-trioxatridecyl]carbamoyl}ethyl)phenoxy]cyclohex-1-en-3-ylidene)ethylidene]-1-(2-sulfonatoethyl)-2,3-dihydro-1H-indole-5-sulfonate, internal salt (Formula XXXII)

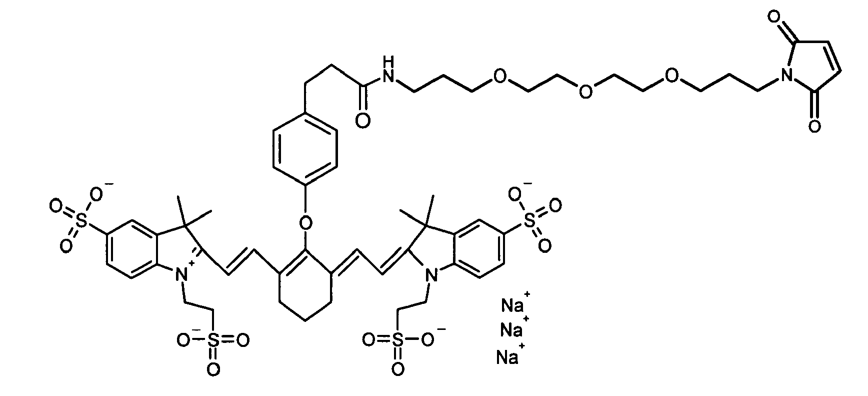

The synthesis is carried out analogously to Example 1e) from 0.7 g (0.68 mmol) of the title compound of Example 14a) and 0.59 g (1.36 mmol) of N-(13-amino-4,7,10-trioxatridecyl)maleimide-trifluoroacetate. Two chromatographic purifications are carried out. Yield: 0.67 g of a blue lyophilizate (75% of theory).

| | | | | | | |
|---|---|---|---|---|---|---|
| Elementary analysis: | Cld.: | C 52.29 | H 5.16 | N 4.28 | S 9.79 | Na 5.27 |
| | Fnd.: | C 51.88 | H 5.40 | N 4.34 | S 9.53 | Na 5.68 |

### Example 16: Trisodium 3,3-dimethyl-2-[2-(1-1{[3,3-dimethyl-5-sulfonato-1-(2-sulfonatoethyl)-3H-indolium-2-yl]vinylene}-2-[4-(2-{[2-(2,5-dioxo-2,5-dihydro-1H-pyrrol-1-yl)ethyl]carbamoyl}ethyl)-phenoxy]-5-tert-butyl-cyclohex-1-en-3-yliden)ethylidene]-1-(2-sulfonatoethyl)-2,3-dihydro-1H-indole-5-sulfonate, internal salt (Formula XXXIII)

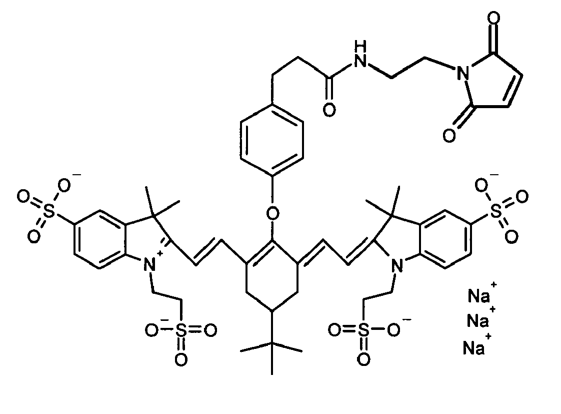

### a) N-[(3-(Anilinomethylene)-2-chloro-5-tert-butyl-1-cyclohexen-1-yl)methylene]aniline hydrochloride

6.7 ml (73.4 mmol) of phosphorus oxychloride is added in drops at 0°C to 8 ml of dimethylformamide. Then, a solution of 5.0 g (32.4 mmol) of 4-*tert*-butylcyclohexanone in 30 ml of dichloromethane is added in drops, and the reaction mixture is stirred under reflux for 3 hours. After cooling to 0°C, 6 g (64.8 mmol) of aniline in 5.5 ml of ethanol is slowly added in drops, the mixture is poured onto 200 g of ice, and 5 ml of concentrated hydrochloric acid is added while being stirred. The precipitated solid is filtered off, washed with ether and dried. Yield: 6.8 g (50% of theory) of a red solid.

### b) Trisodium 3,3-dimethyl-2-[2-(1-{[3,3-dimethyl-5-sulibnato-1-(2-sulfbnatoethyl)-3H-indolium-2-yl]vinylene}-2-chloro-5-tert-butylcyclohex-1-en-3-ylidene)ethylidene]-1-(2-sulfonatoethyl)-2,3-dihydro-1H-indole-5-sulfonate, internal salt

5.0 g (14.4 mmol) of 1-(2-sulfonatoethyl)-2,3,3-trimethyl-3H-indolenine-5-sulfonic acid (Example 1a)) and 3.0 g (7.2 mmol) of N-[(3-(anilinomethylene)-2-chloro-5-*tert*-butyl-1-cyclohexen-1-yl)methylene]aniline hydrochloride (Example 16a)) are refluxed together with 2.5 g (30 mmol) of anhydrous sodium acetate in 100 ml of methanol for 1.5 hours, cooled, mixed with 200 ml of diethyl ether and stirred overnight. The precipitate is suctioned off, dried and purified by chromatography (silica gel, gradient: dichloromethane/methanol). Yield: 4.7 g (68% of theory) of a blue solid.

### c) Trisodium 3,3-dimethyl-2-[2-(1-{[3,3-dimethyl-5-sulfonato-1-(2-sulfonatoethyl)-3H-indolium-2-yl]vinylene}-2-[4-(2-carboxyethyl)phenoxy]-5-tert-butylcyclohex-1-en-3-ylidene)ethylidene]-1-(2-sulfonatoethyl)-2,3-dihydro-1H-indole-5-sulfonate, internal salt

The reaction is carried out from 2.0 g (2.1 mmol) of the title compound of Example 16b) as described in Example 13b). Yield: 1.5 g (66% of theory).

### d) Trisodium 3,3-dimethyl-2-[2-(1-{[3,3-dimethyl-5-sulfonato-1-(2-sulfonatoethyl)-3H-indolium-2-yl]vinylene}-2-[4-(2-{[2-(2,5-dioxo-2,5-dihydro-1H-pyrrol-1-yl)ethyl]-carbamoyl}ethyl)-phenoxy]-5-tert-butyl-cyclohex-1-en-3-ylidene)ethylidene]-1-(2-sulfonatoethyl)-2,3-dihydro-1H-indole-5-sulfonate, internal salt

The reaction is carried out from 1.0 g (0.92 mmol) of the title compound of Example 16c) as described in Example 13c). The purification by chromatography is carried out twice with RP C-18 silica gel (mobile solvent: acetonitrile/water). Yield: 0.24 g (22% of theory).

| | | | | | | |
|---|---|---|---|---|---|---|
| Elementary analysis: | Cld.: | C 52.82 | H 4.93 | N 4.65 | S 10.64 | Na 5.72 |
| | Fnd.: | C 52.23 | H 5.20 | N 4.31 | S 10.30 | Na 6.15 |

### Examples 17 - 19: Synthesis of Indotricarbocyanine Dyes with Bromoacetylamide Groups

### Example 17: Trisodium 3,3-dimethyl-2-{7-[3,3-dimethyl-5-sulfonato-1-(2-sulfonatoethyl)-3H-indolium-2-yl]-4-(5-{[6-(bromoacetylamino)hexyl]carbamoyl}-4-oxapentyl)hepta-2,4,6-trien-1-ylidene}-1-(2-sulfonatoethyl)-2,3-dihydro-1H-indole-5-sulfonate, internal salt (Formula XXXIV)

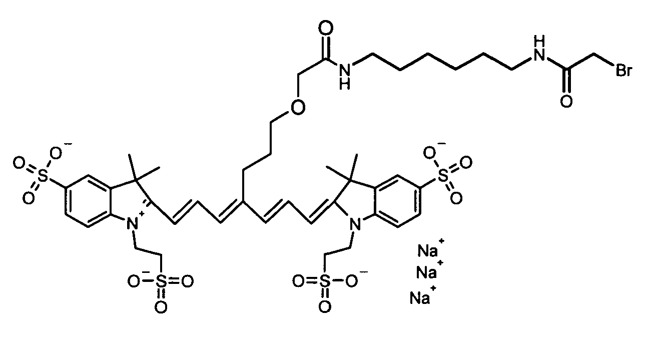

### a) Trisodium 3,3-dimethyl-2-{7-[3,3-dimethyl-5-sulfonato-1-(2-sulfonatoethyl)-3H-indolium-2-yl]-4-(5- {(6-aminohexyl)carbamoyl }-4-oxapentyl)hepta-2,4,6-trien-1-ylidene} -1-(2-sulfonatoethyl)-2,3-dihydro-1H-indole-5-sulfonate, internal salt

The synthesis is carried out analogously to Example 1e) from 0.5 g (0.55 mmol) of the title compound of Example 10c) and 0.15 g (0.70 mmol) of N-boc-hexanediamine (*Fluka*). The reaction product is purified by chromatography (RP C18-chromatography, gradient: methanol/water) and after freeze-drying, it is stirred in 2 ml of trifluoroacetic acid/8 ml of dichloromethane for 15 minutes while being cooled with ice. After spinning-in in a vacuum, the residue is dissolved in methanol, precipitated with diethyl ether and isolated. Yield: 0.26 g of a blue solid (41% of theory).

### b) Trisodium 3,3-dimethyl-2-{7-[3,3-dimethyl-5-sulfonato-1-(2-sulfonatoethyl)-3H-indolium-2-yl]-4-(5-{[6-(bromoacetylamino)hexyl]carbamoyl}-4-oxapentyl)hepta-2,4,6-trien-1-ylidene}-1-(2-sulfonatoethyl)-2,3-dihydro-1H-indole-5-sulfonate, internal salt

0.26 g (0.23 mmol) of the title compound of Example 18a) is cooled in 5 ml of dimethylformamide to -20°C, mixed with 28 mg (0.28 mmol) of triethylamine and a solution of 0.10 g (0.46 mmol) of bromoacetyl bromide in 0.2 ml of dimethylformamide. After 5 hours of stirring at a maximum of 0°C, the product is precipitated by adding diethyl ether and obtained by repeated re-precipitation from dimethylformamide/diethyl ether and subsequent drying. Yield: 0.23 g (86% of theory) of a blue solid.

| | | | | | | |
|---|---|---|---|---|---|---|
| Elementary analysis: | Cld.: | C 45.63 | H 4.87 | N 4.84 | S 11.07 | Na 5.96 |
| | Fnd.: | C 45.13 | H 4.66 | N 4.67 | S 10.83 | Na not determined |

### Example 18: Trisodium 3,3-dimethyl-2-{7-[3,3-dimethyl-5-sulfonato-1-(2-sulfonatoethyl)-3H-indolium-2-yl]-4-(3-{[3-(bromoacetylamino)propyl]carbamoyl}-ethyl)hepta-2,4,6-trien-1-ylidene}-1-(2-sulfonatoethyl)-2,3-dihydro-1H-indole-5-sulfonate, internal salt (Formula XXXV)

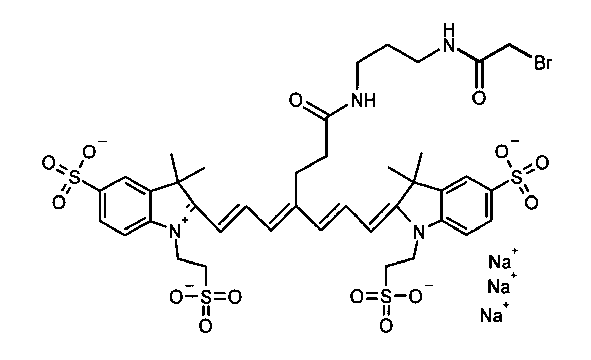

The synthesis is carried out starting from the title compound of Example 1d) (0.5 g; 0.56 mmol) and N-boc-propylenediamine analogously to Example 17. Yield over all the stages: 0.22 g (37% of theory).

| | | | | | | |
|---|---|---|---|---|---|---|
| Elementary analysis: | Cld.: | C 43.70 | H 4.33 | N 5.23 | S 11.96 | Na 6.43 |
| | Fnd.: | C 43.21 | H 4.14 | N 5.53 | S 10.89 | Na not determined |

### Example 19: Trisodium 3,3-dimethyl-2-[2-(1-{[3,3-dimethyl-5-sulfonato-1-(2-sulfonatoethyl)-3H-indolium-2-yl]vinylene}-2-[4-(2-{[3-(bromoacetylamino)propyl]carbamoyl}ethyl)-phenoxy]cyclohex-1-en-3-ylidene)ethylidene]-1-(2-sulfonatoethyl)-2,3-dihydro-1H-indole-5-sulfonate, internal salt (Formula XXXVI)

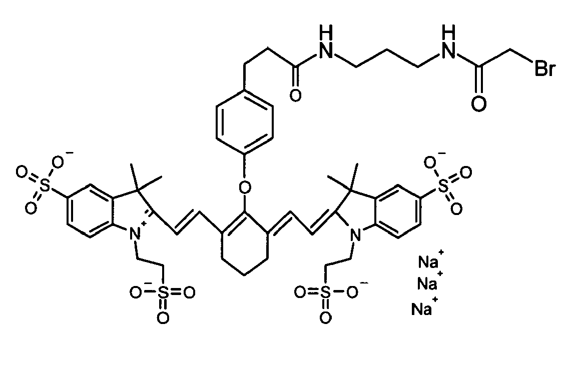

The synthesis is carried out starting from the title compound of Example 13b) (0.5 g; 0.49 mmol) and N-boc-propylenediamine analogously to Example 17. Yield over all stages: 0.31 g (53% of theory).

| | | | | | | |
|---|---|---|---|---|---|---|
| Elementary analysis: | Cld.: | C 47.88 | H 4.52 | N 4.65 | S 10.65 | Na 5.73 |
| | Fnd.: | C 48.04 | H 4.43 | N 4.69 | S 10.72 | Na 5.84 |

### Examples 20-23: Synthesis of conjugates with biomolecules and photophysical characterization of the conjugates

### Example 20: Labeling of BSA (bovine serum albumin) with the title compounds of Examples 1-16

General instructions: A solution of 5 mg (0.074 µmol) of BSA (*Sigma Company*) in 5 ml of phosphate buffer (0.1 M Na₂HPO₄/NaH₂PO₄, pH 6.8) is mixed in each case with 0.74 µmol of the title compounds of Examples 1-16 (stock solutions of 0.5 mg/ml in PBS) and incubated for 30 minutes at 25°C. The purification of the conjugate is carried out by means of gel chromatography (column: Sephadex G50, diameter 1.5 cm, Pharmacia, eluant: PBS).

### Example 21: Labeling of BSA with the title compounds of Examples 17-19

General instructions: A solution of 5 mg (0.074 µmol) of BSA (*Sigma Company*) in 5 ml of phosphate buffer (0.1 M borate buffer, pH 8.5) is mixed in each case with 1.10 µmol of the title compounds of Examples 17-19 (stock solutions of 0.5 mg/ml in PBS) and incubated for 5 hours at 25°C. The purification of the conjugate is carried out by means of gel chromatography (column: Sephadex G50, diameter 1.5 cm, Pharmacia, eluant: PBS).

### Example 22: Labeling of anti-ED-B-fibronectin scFv antibody AP39 (single chain fragment) with the title compounds of Examples 1-16

AP39 is an scFv with a C-terminal cysteine and is present as a covalent S-S-dimer of the molar-mass of about 56,000 g/mol (Curr. Opin. Drug Discov. Devel.. 2002 Mar; 5(2): 204-13). By reduction of the disulfide bridges, two monomers with accessible SH groups are produced (molar mass 28,000 g/mol).

General instructions: 0.3 ml of a solution of AP39 in PBS (conc. 0.93 mg of dimer/ml) is mixed with 60 µl of a solution of tris(carboxyethyl)phosphine (TCEP) in PBS (2.8 mg/ml) and incubated under nitrogen for 1 hour at 25°C. Excess TCEP is separated by means of gel filtration on an NAP-5 column (eluant: PBS). The quantity of AP39-monomer obtained (OD₂₈₀ₙₘ = 1.4), determined by means of photometry, is 230-250 µg (volumes 0.5 - 0.6 ml). The solution is mixed with 0.03 µmol of the title compounds of Examples 1-16 (stock solutions of 0.5 mg/ml in PBS) and incubated for 30 minutes at 25°C. The conjugate is purified by gel chromatography on an NAP-5 column (eluant: PBS/10% glycerol). The immune reactivity of the conjugate solution is determined by means of affinity chromatography (ED-B-fibronectin resin) (J. Immunol. Meth. 1999, 231, 239). The immune reactivity of the conjugates obtained was >80% (AP39 before the conjugation >95%).

### Example 23: Labeling of anti-ED-B-fibronectin scFv antibodies AP39 (single chain fragment) with the title compounds of Examples 17-19

General instructions: 0.3 ml of a solution of AP39 in PBS (conc. 0.93 mg of dimer/ml) is mixed with 60 µl of a solution of tris(carboxyethyl)phosphine (TCEP) in PBS (2.8 mg/ml) and incubated under nitrogen for 1 hour at 25°C. Excess TCEP is separated by means of gel filtration on an NAP-5 column (eluant: 50 mmol of borate buffer pH 8.5). The quantity of AP39-monomer (OD₂₈₀ₙₘ = 1.4) that is obtained, determined by means of photometry, is

230 - 250 µg (volumes 0.5 - 0.6 ml). The solution is mixed with 0.06 µmol of the title compounds of Examples 17-19 (stock solutions of 0.5 mg/ml in PBS) and incubated for 4 hours at 25°C. The conjugate is purified by gel chromatography on an NAP-5 column (eluant: PBS/10% glycerol). The immune reactivity of the conjugate solution is determined by means of affinity chromatography (ED-B-fibronectin resin) (J. Immunol. Meth. 1999, 231, 239). The immune reactivities of the conjugates that were obtained was >75% (AP39 before the conjugation >95%).

### Example 24: Imaging of micrometastasis in Capan-1 tumor bearing nude mice

Capan-1 tumor cells that were grown subconfluently in culture were trypsinized, centrifuged and resuspended in PBS. After staining with trypan blue and calculation of the cell concentration, the cell suspension was set at a concentration of 3x10⁷/ml. The cell suspension was cooled on ice until it was used. Three female nude mice (NMRI-nude, 24-25 g body weight) were anesthetized, and 30 µl (1x10⁶ cells/animal) of the cell suspension inoculated subcapsularly in the pancreas in each animal after abdominal incision. Each animal received 0.05 µmol/kg body weight (1.3 mg/kg body weight) of a substance comprising a cyanine dye according to Example 10, i.e. having a structure as depicted in formula XXVII, which had been conjugated to the EB-DF antibody AP39 according to the method of Example 22. This substance was administered intravenously at a time point that a clear tumor growth was palpable (about 12 to 14 weeks post tumor cell implantation). The animals were sacrificed 6 hours after substance administration and the mesenterium containing micrometastasis was imaged *ex vivo* for fluorescence signals using an intensified CCD camera. The fluorescence of the substance was excited by mesenterium irradiation with near-infrared ligth with 740 nm wavelength, which was produced with a laser diode (0.5 W output). The fluorescence images were stored digitally. Following, the size of micrometastasis were evaluated using a low magnification microscope (Stemi 2000-C, Fa. Carl Zeis). Fluorescence signals were received from micrometastasis in the range of 0.5 to 2.0 mm in diameter and from larger mesenterial metastasis and corresponds with the microscopic evaluation. The effectiveness of the dye conjugates is depicted in Figure 1 based on an example.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

In the foregoing and in the following examples, all temperatures are set forth uncorrected in degrees Celsius, and all parts and percentages are by weight, unless otherwise indicated.

The entire disclosure[s] of all applications, patents and publications, cited herein are incorporated by reference herein.

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make various changes and modifications of the invention to adapt it to various usages and conditions.

## Claims

1. Use of a conjugate of the general formula (I):
B-(D)ₙ (I),
wherein
B stands for an angiogenesis specific binding component,
D stands for a cyanine dye, and
n is 1 to 5
for the production of a diagnostic for the diagnosis of micrometastasis or small proliferative lesions.

2. Use according to claim 1, wherein the angiogenesis specific binding component is directed against the ED-B domain of fibronectin (ED-BF), endoglin, vascular endothelial growth factor receptor (VEGFR), VEGF family members, NRP-1, Ang1, Thie2, PDGF-BB and receptors, TGF-β1, endoglin, TGF-β receptors, FGF, HGF, MCP-1, Integrins (αᵥβ₃, αᵥβ₅, α₅β₁), VE-cadherin, PECAM (CD31), Ephrins, Plasminogen activators, MMPs, PAI-1, NOS, COX-2, AC133, Chemokins, Id1/Id3, VEGFR-1, Ang2, TSP-1,-2, Angiostatin and related plasminogen kringles, Endostatin (collagen XVII fragment), Vasostatin, Platelet factor 4, TIMPs, MMP inhibitors, PEX, Meth-1, Meth-2, IFN-α, -β, -γ, IP-10, IL-4, IL-12, IL-18, Prolactin (M, 16K), VEGI, Fragment of SPARC, Osteopontin fragment or Maspin.

3. Use according to claim 1 or 2, wherein the angiogenesis specific binding component is selected from the group consisting of a peptide, a protein, a nucleic acid, a small molecule, and a sugar.

4. Use according to one of claims 1 to 3, wherein the protein is selected from the group consisting of an antibody, an antibody fragment, and a single chain antibody.

5. Use according to one of claims 1 to 4, wherein the antibody is selected from the group consisting of L19, E8, AP38 and AP39.

6. Use according to one of claims 1 to 5, wherein the nucleic acid is selected from the group consisting of DNA, RNA, aptamers, and PNA.

7. Use according to one of claims 1 to 6, wherein the small molecule is 2,2-diphenylethylamine.

8. Use according to one of claims 1 to 7, wherein the cyanine dye is selected from the group consisting of carbocyanine, dicarbocyanine, and tricarbocyanine.

9. Use according to one of claims 1 to 6, wherein the cyanine dye has the general formula (II) 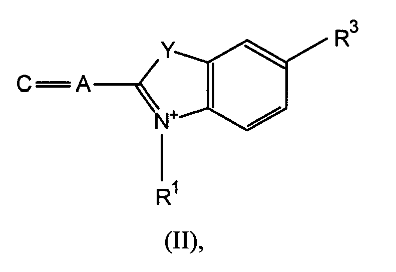 wherein C stands for a radical (III) or (IV) 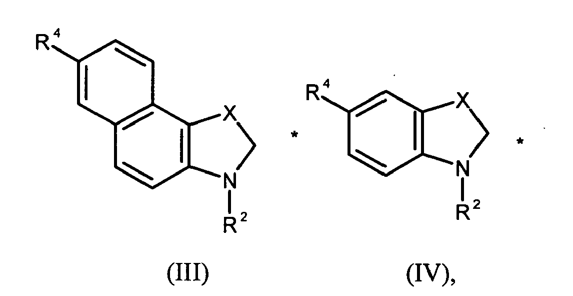 wherein the position that is labeled with the star means the point of linkage with radical
A and can stand for the group (V), (VI), (VII), (VIII) or (IX) 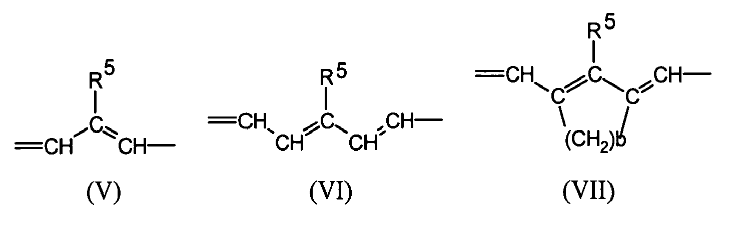 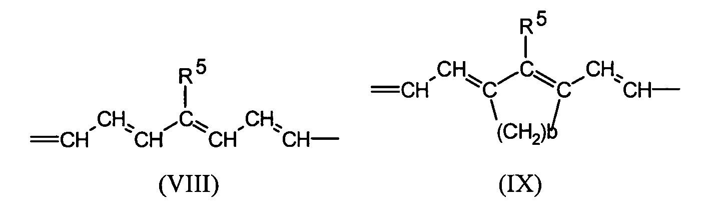 wherein
R¹ and R², independently of one another, stand for a C₁-C₄-sulfoalkyl chain or a saturated or unsaturated, branched or straight-chain C₁-C₅₀-alkyl chain, which optionally is substituted by 0 to 15 oxygen atoms and/or by 0 to 3 carbonyl groups and/or with 0 to 5 hydroxyl groups or optionally interrupted by 0 to 15 oxygen atoms and/or by 0 to 3 carbonyl groups and/or can be substituted with 0 to 5 hydroxyl groups;
R³ stands for B or a linker connected to B, wherein the linker is a branched or straight-chain carbohydrate chain with up to 20 carbon residues, which is substituted with one or more -OH, -COOH, -SO₃ groups and/or optionally interrupted one or more times by -O-, -S-, -CO-, -CS-, -CONH, -NHCO-, NHCSNH-, -SO₂-, -PO₄⁻-, -aryl- and/or -NH- group;
R⁴ stands for the group -COOE¹, -CONE¹E², -NHCOE¹, -NHCONHE¹, -NE¹E², - OE¹, -OSO₃E¹, -SO₃E¹, -SO₂NHE¹ or -E¹, wherein
E¹ and E², independently of one another, stand for a hydrogen atom, a C₁-C₄-sulfoalkyl chain, a saturated or unsaturated, branched or straight-chain C₁-C₅₀-alkyl chain, which optionally is interrupted by 0 to 15 oxygen atoms and/or by 0 to 3 carbonyl groups and/or is substituted with 0 to 5 hydroxyl groups;
R⁵ stands for a hydrogen atom, or a fluorine, chlorine, bromine or iodine atom, methyl, ethyl, propyl or *iso*-propyl;
b means the number 2 or 3; and
X and Y, independently of one another, stand for O, S, =C(CH₃)₂ or-(CH=CH)-,
as well as salts and solvates of these compounds.

10. Use according to one of claims 1 to 8, wherein the cyanine dye has the general formula (X) 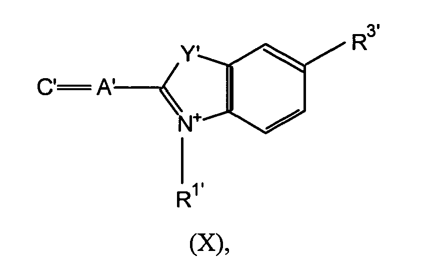 wherein C' stands for a radical (XI) or (XII) 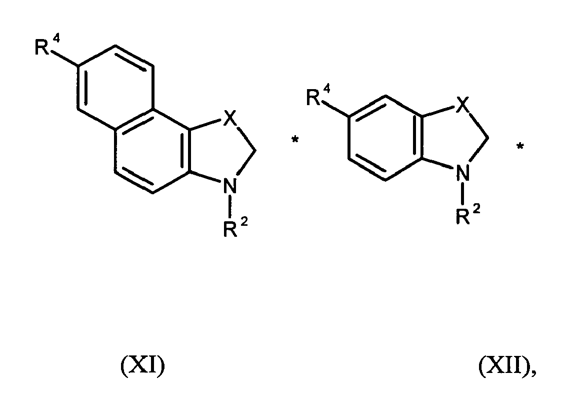 wherein the position that is labeled with the star means the point of linkage with radical
A' and can stand for the group (XIII), (XIV), (XV), (XVI) or (XVII) 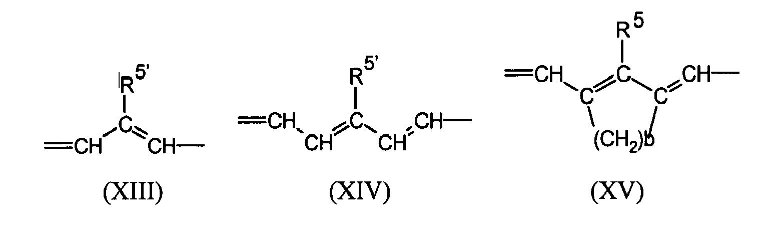 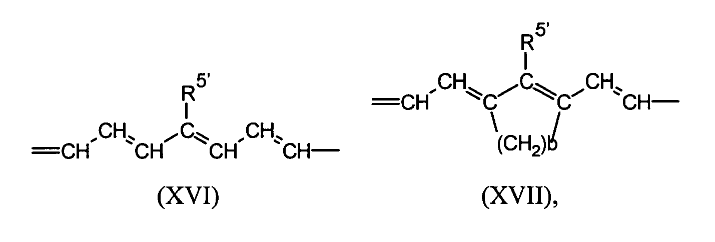 wherein radical (XV) or (XVII) optionally can be substituted with a C₁ to C₄-alkyl radical,
wherein
R^{1'} stands for a C₁-C₄-sulfoalkyl chain; a saturated or unsaturated, branched or straight-chain C₁-C₅₀-alkyl chain, which optionally is substituted by 0 to 15 oxygen atoms and/or by 0 to 3 carbonyl groups and/or can be substituted with 0 to 5 hydroxyl groups or optionally interrupted by 0 to 15 oxygen atoms and/or by 0 to 3 carbonyl groups and/or can be substituted with 0 to 5 hydroxyl groups; or M'-R^{6'};
R^{2'} stands for a C₁-C₄-sulfoalkyl chain; a saturated or unsaturated, branched or straight-chain C₁-C₅₀-alkyl chain, which optionally is substituted by 0 to 15 oxygen atoms and/or by 0 to 3 carbonyl groups and/or can be substituted with 0 to 5 hydroxyl groups or optionally interrupted by 0 to 15 oxygen atoms and/or by 0 to 3 carbonyl groups and/or can be substituted with 0 to 5 hydroxyl groups; or M'-R^{7'};
R^{3'}, R^{4'}, R^{6'} and R^{7'}, independently of one another, stand for the group -COOE^{1'}, - CONE^{1'}E^{2'}, -NHCOE^{1'}, -NHCONHE^{1'}, -NE^{1'}E^{2'} -OE^{1'} -OSO₃E^{1'}, -SO₃E^{1'}, - SO₂NHE^{1'} or -E^{1'}, wherein
E^{1'} and E^{2'}, independently of one another, stand for a hydrogen atom, a C₁-C₄-sulfoalkyl chain, a saturated or unsaturated, branched or straight-chain C₁-C₅₀-alkyl chain, which optionally is interrupted by 0 to 15 oxygen atoms and/or by 0 to 3 carbonyl groups and/or is substituted with 0 to 5 hydroxyl groups;
M' stands for CH₂-CH₂ or CH₂-CH₂-CH₂;
R^{5'} stands for -Q'-CH₂-R^{8'};
Q' stands for C₁ to C₅ alkyl, whereby the C atoms are optionally substituted by O or S, or stands for 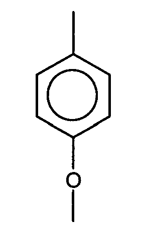
R^{8'} stands for -CO-NH-R^{9'}-R^{10'}, -NH-CS-NH- R^{9'}-R^{10'} or -NH-CO- R^{9'}-R^{10'},
wherein
R^{9'} is selected from the group consisting of unbranched C₂-C₁₃ alkyl, in which C atoms are optionally replaced by O or S, and
R^{10'} is B or the residual part of a coupling moiety, which is linked to B, and
b' means the number 2 or 3; and
X' and Y', independently of one another, stand for O, S, =C(CH₃)₂ , =C(C₂H₅)₂, =C(C₃H₇)₂, =C(*iso*C₃H₇)₂, =C(C₄H₉)₂, or -(CH=CH)-,
as well as salts and solvates of these compounds.

11. Use according to claim 10, wherein
A' stands for a radical (XVI) or (XVII), wherein radical (XVII) optionally can be substituted in para-position with a C₁ to C₄-alkyl radical;
C' stands for a radical (XII);
R^{1'} stands for M-R^{6'};
R^{2'} stands for M-R^{7'};
R^{3'}, R^{4'}, R^{6'}, and R^{7'}, independently of one another, stand for SO₃H or H, with the proviso that at least three of R^{3'}, R^{4'}, R^{6'}, and R^{7'} are SO₃H, and
X' and Y', independently of one another, stand for O, S, =C(CH₃)₂ , =C(C₂H₅)₂, =C(C₃H₇)₂, =C(*iso*C₃H₇)₂, or =C(C₄H₉)₂,
b' is 3.

12. Use according to claims 10 or 11, wherein
A' stands for the radical with the formula (XVI);
M' stands for CH₂-CH₂; and
Q' stands for C₁ to C₅ alkyl, whereby the C atoms are optionally substituted by O or S.

13. Use according to one of claims 10 to 12, wherein
Q' stands for C₁-C₅ alkyl.

14. Use according to claim 10 or 11, wherein
A' stands for the radical with the formula (XVII)
b' means 3, and
Q' stands for

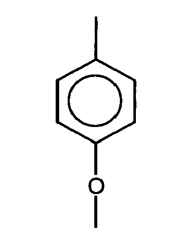

15. Use according to one of claims 10 to 14, wherein
R^{8'} stands for CO-B or NH-B.

16. Use according to one of claims 1 to 15, wherein the early stage proliferative disease is selected from the group consisting of a small primary tumor, a precancerosis, a dysplasia, a metaplasia, psoriasis, psoriatic arthritis, rheumatoid arthritis, endometriosis and/or an ocular disease.

17. Use according to one of claims 1 to 16, for the *in vivo* diagnosis.

18. Use according to one of claims 1 to 17, wherein the micrometastasis and/or the small proliferative lesion is diagnosed prior, during and/or after a treatment procedure.

19. Use according to one of claims 1 to 18, wherein the micrometastasis and/or the small proliferative lesion has a diameter of less than 10 mm, preferably of less than 8 mm.

20. Use according to one of claims 1 to 18, wherein the micrometastasis and/or the small proliferative lesion has a diameter of less than 6 mm, preferably of less than 5 mm.

21. Use according to one of claims 1 to 18, wherein the micrometastasis and/or the small proliferative lesion has a diameter of less than 4 mm, preferably of less than 3 mm.

22. Use according to one of claims 1 to 18, wherein the micrometastasis and/or the small proliferative lesion has a diameter of between 2.0 to 0.2 mm.

23. Use according to one of claims 1 to 22, wherein the micrometastasis is an iatrogenic micrometastasis, a hematogenous micrometastasis, a cavitary micrometastasis, an intraluminal micrometastasis, a lymphatic metastasis, a local micrometastasis, and/or a regional micrometastasis.

24. Use according to one of claims 1 to 22, wherein the precancerosis is selected from the group consisting of precancerosis of the skin, in particular actinic keratosis, cutaneaous horn, actinic cheilitis, tar keratosis, arsenic keratosis, x-ray keratosis, Bowen's disease, bowenoid papulosis, lentigo maligna, lichen sclerosus, and lichen rubber mucosae; precancerosis of the digestive tract, in particular erythroplakia, leukoplakia, Barrett's esophagus, Plummer-Vinson syndrome, crural ulcer, gastropathia hypertrophica gigantea, borderline carcinoma, neoplastic intestinal polyp, rectal polyp, porcelain gallbladder; gynaecological precancerosis, in particular carcinoma ductale in situ (CDIS), cervical intraepithelial neoplasia (CIN), leukoplakia, endometrial hyperplasia (grade III), vulvar dystrophy, vulvar intraepithelial neoplasia (VIN), hydatidiform mole; urologic precancerosis, in particular bladder papillomatosis, Queyrat's erythroplasia, testicular intraepithelial neoplasia (TIN), leukoplakia; carcinoma in situ (CIS); precancerosis caused by chronic inflammation, in particular pyoderma, osteomyelitis, acne conglobata, lupus vulgaris, and fistula.

25. Use according to one of claims 1 to 22, wherein the metaplasia is selected from the group consisting of agnogenic myeloid metaplasia, apocrine metaplasia, atypical metaplasia, autoparenchymatous metaplasia, connective tissue metaplasia, epithelial metaplasia, intestinal metaplasia, metaplastic anemia, metaplastic ossification, metaplastic polyps, myeloid metaplasia, primary myeloid metaplasia, secondary myeloid metaplasia, squamous metaplasia, squamous metaplasia of amnion, symptomatic myeloid metaplasia and regenerative metaplasia.

26. Use according to one of claims 1 to 22, wherein the dysplasia is selected from the group consisting of anhidrotic ectodermal dysplasia, anterofacial dysplasia, asphyxiating thoracic dysplasia, atriodigital dysplasia, bronchopulmonary dysplasia, cerebral dysplasia, cervical dysplasia, chondroectodermal dysplasia, cleidocranial dysplasia, congenital ectodermal dysplasia, craniodiaphysial dysplasia, craniocarpotarsal dysplasia, craniometaphysial dysplasia, dentin dysplasia, diaphysial dysplasia, ectodermal dysplasia, enamel dysplasia, encephalo-ophthalmic dysplasia, dysplasia epiphysialis heminelia, dysplasia epiphysialis multiplex, dysplasia epiphysalis punctata, epithelial dysplasia, faciodigitogenital dysplasia, familial fibrous dysplasia of jaws, familial white folded dysplasia, fibromuscular dysplasia, fibrous dysplasia of bone, florid osseous dysplasia, hereditary renal-retinal dysplasia hidrotic ectodermal dysplasia, hypohidrotic ectodermal dysplasia, lymphopenic thymic dysplasia, mammary dysplasia, mandibulofacial dysplasia, metaphysical dysplasia, Mondini dysplasia, monostotic fibrous dysplasia, mucoepithelial dysplasia, multiple epiphysial dysplasia, oculoauriculovertebral dysplasia, oculodentodigital dysplasia, oculovertebral dysplasia, odontogenic dysplasia, ophthalmomandibulomelic dysplasia, periapical cemental dysplasia, polyostotic fibrous dysplasia, pseudoachondroplastic spondyloepiphysial dysplasia, retinal dysplasia, septo-optic dysplasia, spondyloepiphysial dysplasia, and ventriculoradial dysplasia.

27. Use according to one of claims 1 to 22, wherein the endometriosis comprises hematogenous cell clusters, cavitary cell clusters, intraluminal cell clusters, lymphatic cell clusters, local cell clusters and/or regional cell clusters.

28. Use according to one of claims 1 to 22, wherein the ocular disease is selected from the group consisting of trachoma, retinopathy of prematurity, diabetic retinopathy, neovascular glaucoma and age-related macular degeneration.
